(19) Europäisches Patentamt
European Patent Office
Office européen des brevets

(11) Veröffentlichungsnummer: **0 424 317 A2**

# (12) EUROPÄISCHE PATENTANMELDUNG

(21) Anmeldenummer: **90810777.4**

(22) Anmeldetag: **10.10.90**

(51) Int. Cl.5: **C07D 403/10**, C07D 239/34, A61K 31/505

(30) Priorität: **19.10.89 CH 3799/89**

(43) Veröffentlichungstag der Anmeldung: **24.04.91 Patentblatt 91/17**

(84) Benannte Vertragsstaaten: **AT BE CH DE DK ES FR GB GR IT LI LU NL SE**

(71) Anmelder: **CIBA-GEIGY AG**
**Klybeckstrasse 141**
**CH-4002 Basel(CH)**

(72) Erfinder: **Herold, Peter, Dr.**
**Mattweg 19**
**CH-4144 Arlesheim(CH)**
Erfinder: **Bühlmayer, Peter, Dr.**
**Hangstrasse 18**
**CH-4144 Arlesheim(CH)**

## (54) Pyrimidine.

(57) Pyrimidin-Verbindungen der Formel

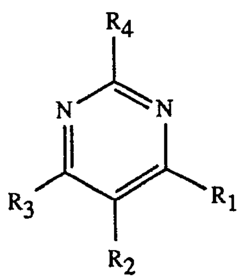

(I),

worin einer der Reste $R_1$, $R_2$ und $R_3$ einen gegebenenfalls durch Halogen oder Hydroxy substituierten aliphatischen Kohlenwasserstcffrest oder einen cycloaliphatischen oder araliphatischen Kohlenwasserstoffrest bedeutet, der zweite der Reste $R_1$, $R_2$ und $R_3$ und der Rest $R_4$ jeweils unabhängig voneinander Halogen, Acyl, einen aromatischen Kohlenwasserstoffrest, gegebenenfalls verestertes oder amidiertes Carboxy, Cyano, $SO_3H$, $PO_2H_2$, $PO_3H_2$, 5-Tetrazolyl, gegebenenfalls substituiertes Sulfamoyl, Acylamino oder $-Z_1-R'$, worin $Z_1$ für eine Bindung oder für O, $S(O)_m$ oder N(R) steht, $R'$ Wasserstoff oder einen aliphatischen Kohlenwasserstoffrest bedeutet, der gegebenenfalls durch O oder $S(O)_m$ unterbrochen ist und gegebenenfalls durch Halogen, Hydroxy, gegebenenfalls substituiertes Amino oder gegebenenfalls verestertes oder amidiertes Carboxy substituiert ist, R Wasserstoff oder einen aliphatischen Kohlenwasserstoffrest bedeutet und m jeweils für 0,1 oder 2 steht, bedeuten und der dritte der Reste $R_1$, $R_2$ und $R_3$ für die Gruppe der Formel

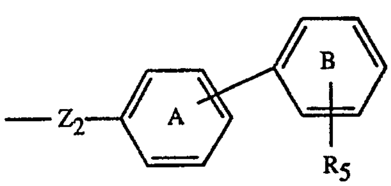

(Ia)

EP 0 424 317 A2

steht, worin $Z_2$ für Alkylen, O, $S(O)_m$ oder N(R) steht, $R_5$ Carboxy, Halogenalkansulfonylamino, $SO_3H$, $PO_2H_2$, $PO_3H_2$ oder 5-Tetrazolyl bedeutet, R Wasserstoff oder einen aliphatischen Kohlenwasserstoffrest bedeutet, m für 0,1 oder 2 steht und die Ringe A und B unabhängig voneinander gegebenenfalls durch Halogen, einen aliphatischen Kohlenwasserstoffrest, der gegebenenfalls durch O unterbrochen ist und gegebenenfalls durch Hydroxy oder Halogen substituiert ist, gegebenenfalls durch einen aliphatischen Alkohol verethertes Hydroxy, gegebenenfalls verestertes oder amidiertes Carboxy oder 5-Tetrazolyl substituiert sind, und gegebenenfalls Tautomere davon, jeweils in freier Form oder in Salzform, sind in an sich bekannter Weise herstellbar und können beispielsweise als Arzneimittelwirkstoffe verwendet werden.

# PYRIMIDINE

Die Erfindung betrifft Pyrimidin-Verbindungen der Formel

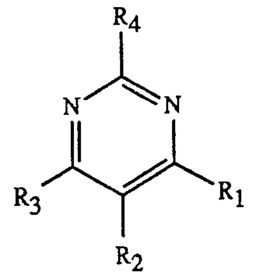

(I),

worin einer der Reste $R_1$, $R_2$ und $R_3$ einen gegebenenfalls durch Halogen oder Hydroxy substituierten aliphatischen Kohlenwasserstoffrest oder einen cycloaliphatischen oder araliphatischen Kohlenwasserstoffrest bedeutet, der zweite der Reste $R_1$, $R_2$ und $R_3$ und der Rest $R_4$ jeweils unabhängig voneinander Halogen, Acyl, einen aromatischen Kohlenwasserstoffrest, gegebenenfalls verestertes oder amidiertes Carboxy, Cyano, $SO_3H$, $PO_2H_2$, $PO_3H_2$, 5-Tetrazolyl, gegebenenfalls substituiertes Sulfamoyl, Acylamino oder $-Z_1-R'$ ,worin $Z_1$ für eine Bindung oder für O, $S(O)_m$ oder N(R) steht, $R'$ Wasserstoff oder einen aliphatischen Kohlenwasserstoffrest bedeutet, der gegebenenfalls durch O oder $S(O)_m$ unterbrochen ist und gegebenenfalls durch Halogen, Hydroxy, gegebenenfalls substituiertes Amino oder gegebenenfalls verestertes oder amidiertes Carboxy substituiert ist, R Wasserstoff oder einen aliphatischen Kohlenwasserstoffrest bedeutet und m jeweils für 0,1 oder 2 steht, bedeuten und der dritte der Reste $R_1$, $R_2$ und $R_3$ für die Gruppe der Formel

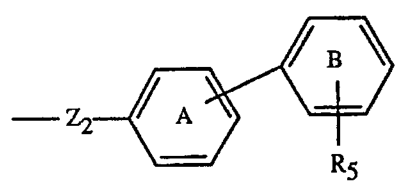

(Ia)

steht, worin $Z_2$ für Alkylen, O, $S(O)_m$ oder N(R) steht, $R_5$ Carboxy, Halogenalkansulfonylamino, $SO_3H$, $PO_2H_2$, $PO_3H_2$ oder 5-Tetrazolyl bedeutet, R Wasserstoff oder einen aliphatischen Kohlenwasserstoffrest bedeutet, m für 0,1 oder 2 steht und die Ringe A und B unabhängig voneinander gegebenenfalls durch Halogen, einen aliphatischen Kohlenwasserstoffrest, der gegebenenfalls durch O unterbrochen ist und gegebenenfalls durch Hydroxy oder Halogen substituiert ist, gegebenenfalls durch einen aliphatischen Alkohol verethertes Hydroxy, gegebenenfalls verestertes oder amidiertes Carboxy oder 5-Tetrazolyl substituiert sind, und gegebenenfalls Tautomere davon, jeweils in freier Form oder in Salzform, ein Verfahren zur Herstellung dieser Verbindungen und Tautomere, die Verwendung dieser Verbindungen und Tautomere und pharmazeutische Präparate, enthaltend eine solche Verbindung I oder ein solches Tautomeres, jeweils in freier Form oder in Form eines pharmazeutisch verwendbaren Salzes.

Die Verbindungen I können teilweise als Protonentautomere vorliegen. Bedeutet z. B. einer der Reste $R_1$, $R_2$ und $R_3$ Hydroxy, können entsprechende Verbindungen im Gleichgewicht mit den tautomeren Oxo-Derivaten, d. h. den entsprechenden Pyrimid-4-onen, -5-onen oder -6-onen, stehen. Entsprechend sind unter den Verbindungen I vor- und nachstehend gegebenenfalls auch entsprechende Tautomere zu verstehen.

Die Verbindungen der Formel I und gegebenenfalls ihre Tautomeren können als, insbesondere pharmazeutisch verwendbare, Salze vorliegen. Weisen die Verbindungen I z. B. mindestens ein basisches Zentrum auf, können sie Säureadditionssalze bilden. Diese werden beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie gegebenenfalls, z.B. durch Halogen, substituierten $C_1$-$C_4$-Alkancarbonsäuren, z.B. Essigsäure, wie gegebenenfalls ungesättigten Dicarbonsäuren, z.B. Oxal-, Malon-, Bernstein-, Malein-, Fumar-, Phthal- oder Terephthalsäure, wie Hydroxycarbonsäuren, z.B. Ascorbin-, Glykol-, Milch-, Äpfel-, Wein- oder Zitronensäure, wie Aminosäuren, z.B. Asparagin- oder

Glutaminsäure, oder wie Benzoesäure, oder mit organischen Sulfonsäuren, wie gegebenenfalls, z.B. durch Halogen, substituierten $C_1$-$C_4$-Alkan- oder Aryl-sulfonsäuren, z.B. Methan- oder p-Toluolsulfonsäure, gebildet. Entsprechende Säureadditionssalze können auch mit einem gegebenenfalls zusätzlich vorhandenen basischen Zentrum gebildet werden. Ferner können die Verbindungen I mit mindestens einer aciden Gruppe (beispielsweise COOH oder 5-Tetrazolyl) Salze mit Basen bilden. Geeignete Salze mit Basen sind beispielsweise Metallsalze, wie Alkali- oder Erdalkalimetallsalze, z.B. Natrium-, Kalium- oder Magnesiumsalze, oder Salze mit Ammoniak oder einem organischen Amin, wie Morpholin, Thiomorpholin, Piperidin, Pyrrolidin, einem Mono-, Di- oder Triniederalkylamin, z. B. Ethyl-, tert.-Butyl-, Diethyl-, Diisopropyl-, Triethyl-, Tributyl- oder Dimethyl-propyl-amin, oder einem Mono-, Di- oder Trihydroxyniederalkylamin, z.B. Mono-, Di- oder Triethanolamin. Weiterhin können entsprechende innere Salze gebildet werden. Umfasst sind ferner für pharmazeutische Verwendungen nicht geeignete Salze, die beispielsweise für die Isolierung bzw. Reinigung von freien Verbindungen I oder deren pharmazeutisch verwendbaren Salzen eingesetzt werden.

Ein aromatischer Kohlenwasserstoffrest ist insbesondere Phenyl.

Acyl bedeutet insbesondere Niederalkanoyl.

Verestertes Carboxy bedeutet beispielsweise Carboxy, welches durch einen aliphatischen Alkohol verestert ist, der sich von einem aliphatischen Kohlenwasserstoffrest ableitet, wie von Niederalkyl, Niederalkenyl oder in zweiter Linie Niederalkinyl, welcher gegebenenfalls durch O unterbrochen ist, wie von Niederalkoxy-niederalkyl, -niederalkenyl oder -niederalkinyl.

Amidiertes Carboxy ist beispielsweise Carbamoyl, in welchem die Aminogruppe gegebenenfalls durch einen aliphatischen oder araliphatischen Kohlenwasserstoffrest, wie Niederalkyl, Niederalkenyl, Niederalkinyl oder Phenyl-niederalkyl, -niederalkenyl oder -niederalkinyl, unabhängig voneinander mono- oder disubstituiert oder durch einen zweiwertigen aliphatischen Kohlenwasserstoffrest, der gegebenenfalls durch O unterbrochen ist, wie Niederalkylen oder Niederalkylenoxyniederalkylen, disubstituiert ist.

Substituiertes Amino ist beispielsweise durch einen aliphatischen oder araliphatischen Kohlenwasserstoffrest, wie Niederalkyl, Niederalkenyl, Niederalkinyl oder Phenyl-niederalkyl, -niederalkenyl oder -niederalkinyl, unabhängig voneinander mono-oder disubstituiertes oder durch einen zweiwertigen aliphatischen Kohlenwasserstoffrest, der gegebenenfalls durch O unterbrochen ist, wie Niederalkylen oder Niederalkylenoxyniederalkylen, disubstituiertes Amino. Als Beispiele seien Niederalkyl-, Niederalkenyl-, Niederalkinyl-, Phenylniederalkyl-, Phenylniederalkenyl-, Phenylniederalkinyl-, Diniederalkyl-, N-Niederalkyl-N-phenylniederalkyl- und Di(phenylniederalkyl)amino genannt.

Ein aliphatischer Kohlenwasserstoffrest ist beispielsweise Niederalkyl, Niederalkenyl oder in zweiter Linie Niederalkinyl.

Ein aliphatischer Kohlenwasserstoffrest, der durch O unterbrochen ist, ist insbesondere Niederalkoxy-niederalkyl, -niederalkenyl oder -niederalkinyl oder Niederalkenyloxy-niederalkyl, -niederalkenyl oder -niederalkinyl, während ein aliphatischer Kohlenwasserstoffrest, der durch $S(O)_m$ unterbrochen ist, insbesondere Niederalkylthio-niederalkyl, -niederalkenyl oder -niederalkinyl, Niederalkan-sulfinylniederalkyl oder -sulfonylniederalkyl, Niederalkenyl-thioniederalkyl, -sulfinylniederalkyl oder -sulfonylniederalkyl oder Niederalkinyl-thioniederalkyl, -sulfinylniederalkyl oder -sulfonylniederalkyl ist.

Ein durch Halogen oder Hydroxy substituierter aliphatischer Kohlenwasserstoffrest ist beispielsweise Halogen-niederalkyl, -niederalkenyl oder -niederalkinyl oder Hydroxy-niederalkyl, -niederalkenyl oder -niederalkinyl.

Ein durch Halogen oder Hydroxy substituierter aliphatischer Kohlenwasserstoffrest, der durch O oder $S(O)_m$ unterbrochen ist, ist ein entsprechender vorstehend angegebener Rest, der durch Halogen oder Hydroxy substituiert ist.

Ein durch gegebenenfalls substituiertes Amino oder gegebenenfalls verestertes oder amidiertes Carboxy substituierter aliphatischer Kohlenwasserstoffrest, der gegebenenfalls durch O oder $S(O)_m$ unterbrochen ist, ist ein entsprechender vorstehend angegebener Rest, der durch Amino, vorstehend angegebenes substituiertes Amino, Carboxy, vorstehend angegebenes verestertes Carboxy oder vorstehend angegebenes amidiertes Carboxy substituiert ist.

Ein cycloaliphatischer Kohlenwasserstoffrest ist beispielsweise Cycloalkyl oder in zweiter Linie Cycloalkenyl.

Als araliphatische Kohlenwasserstoffreste kommen insbesondere Phenylniederalkyl, ferner Phenylniederalkenyl und -niederalkinyl in Frage.

Mit einem aliphatischen Alkohol verethertes Hydroxy ist insbesondere Niederalkoxy oder Niederalkenyloxy.

Alkylen ist Methylen oder Niederalkylen.

Substituiertes Sulfamoyl bedeutet Niederalkyl- oder Diniederalkyl-sulfamoyl.

In Acylamino leitet sich Acyl von einer organischen Carbonsäure oder einer organischen Sulfonsäure ab. Beispielhaft seien als entsprechendes Acyl Niederalkanoyl, gegebenenfalls substituiertes Benzoyl, Niederalkansulfonyl, Halogenniederalkansulfonyl oder gegebenenfalls substituiertes Benzolsulfonyl genannt.

Vor- und nachstehend sind ungesättigte aliphatische, cycloaliphatische und araliphatische Substituenten in erster Linie nicht über ein C-Atom, von dem eine Mehrfachbindung ausgeht, mit einem aromatischen Rest verknüpft.

Phenyl bedeutet jeweils unsubstituiertes oder ein- oder mehrfach, z.B. zwei- oder dreifach, z.B. durch (einen) Substituenten ausgewählt aus der Gruppe bestehend aus Niederalkyl, Niederalkoxy, Halogen, Trifluormethyl und Hydroxy, substituiertes Phenyl. Dies gilt für Phenylreste per se und für Phenyl in Phenyl enthaltenden Gruppen, wie Benzoyl oder Benzolsulfonyl.

Die Ringe A und B bilden einen Biphenylylrest, wobei entsprechendes 4-Biphenylyl bevorzugt ist.

Die vor- und nachstehend verwendeten Allgemeinbegriffe haben, sofern nicht abweichend definiert, folgende Bedeutungen:

Der Ausdruck "Nieder" bedeutet, dass entsprechende Gruppen und Verbindungen jeweils insbesondere bis und mit 7, vorzugsweise bis und mit 4, Kohlenstoffatome enthalten.

Niederalkansulfonyl bedeutet insbesondere $C_1$-$C_7$-Alkansulfonyl und ist z.B. Methan-, Ethan-, n-Propan- oder Isopropansulfonyl. Bevorzugt ist $C_1$-$C_4$-Alkansulfonyl. Halogenniederalkansulfonyl ist Halogen-$C_1$-$C_7$-alkansulfonyl, wie Trifluormethansulfonyl.

Niederalkansulfamoyl ist $C_1$-$C_7$-Alkansulfamoyl, wie Methan-, Ethan-, n-Propan-, Isopropan-, n-Butan-, sek.-Butan- oder tert.-Butansulfamoyl. Bevorzugt ist $C_1$-$C_4$-Alkansulfamoyl. Diniederalkansulfamoyl bedeutet Di-$C_1$-$C_7$-alkansulfamoyl, wie Dimethan-, Methan-ethan- oder Di-(n-propan)-sulfamoyl. Bevorzugt ist Di-$C_1$-$C_4$-alkansulfamoyl.

Halogen ist insbesondere Halogen mit einer Atomnummer bis und mit 35, d. h. Fluor, Chlor oder Brom, und umfasst ferner lod.

Halogenalkansulfonylamino bedeutet insbesondere Halogen-$C_1$-$C_7$-alkansulfonylamino und ist z.B. Difluormethan-, Trifluormethan-, 2-Chlorethan-, 1,1,2-Trifluorethan-, 1,1,2-Trichlorethan-, Pentafluorethan- oder Heptafluorpropan-sulfonylamino. Bevorzugt ist Halogen-$C_1$-$C_4$-alkansulfonylamino.

Niederalkanoyl bedeutet insbesondere $C_1$-$C_7$-Alkanoyl und ist z.B. Formyl, Acetyl, Propionyl, Butyryl, Isobutyryl oder Pivaloyl. Bevorzugt ist $C_2$-$C_5$-Alkanoyl.

Niederalkyl ist insbesondere $C_1$-$C_7$-Alkyl, d. h. Methyl, Ethyl, n-Propyl, Isopropyl, n-Butyl, Isobutyl, sek.-Butyl, tert-Butyl oder ein entsprechender Pentyl-, Hexyl- oder Heptylrest. Bevorzugt ist $C_1$-$C_4$-Alkyl.

Niederalkenyl bedeutet insbesondere $C_3$-$C_7$-Alkenyl und ist z.B. Propen-2-yl, Allyl oder But-1-en-3-yl, -1-en-4-yl, -2-en-1-yl oder -2-en-2-yl. Bevorzugt ist $C_3$-$C_5$-Alkenyl.

Niederalkinyl ist insbesondere $C_3$-$C_7$-Alkinyl und bedeutet vorzugsweise Propargyl.

Niederalkoxy ist insbesondere $C_1$-$C_7$-Alkoxy d. h. Methoxy, Ethoxy, n-Propyloxy, Isopropyloxy, n-Butyloxy, Isobutyloxy, sek.-Butyloxy, tert.-Butyloxy oder entsprechendes Pentyloxy, Hexyloxy oder Heptyloxy. Bevorzugt ist $C_1$-$C_4$-Alkoxy.

Niederalkoxyniederalkyl bedeutet insbesondere $C_1$-$C_4$-Alkoxy-$C_1$-$C_4$-alkyl, wie 2-Methoxyethyl, 2-Ethoxyethyl, 2-(n-Propyloxy)ethyl oder Ethoxymethyl.

Niederalkoxy-niederalkenyl bzw. -niederalkinyl bedeutet insbesondere $C_1$-$C_4$-Alkoxy-$C_3$-$C_5$-alkenyl bzw. -alkinyl.

Niederalkenyloxy bedeutet insbesondere $C_3$-$C_7$-Alkenyloxy und ist z.B. Allyloxy, But-2-en-1-yloxy oder But-3-en-1-yloxy. Bevorzugt ist $C_3$-$C_5$-Alkenyloxy.

Halogenniederalkyl bedeutet insbesondere Halogen-$C_1$-$C_4$-alkyl, wie Trifluormethyl, 1,1,2-Trifluor-2-chlor-ethyl, Chlormethyl oder n-Heptafluorpropyl.

Halogenniederalkenyl bedeutet insbesondere Halogen-$C_3$-$C_5$-alkenyl, wie 3-Chlorallyl.

Halogenniederalkinyl ist insbesondere Halogen-$C_3$-$C_5$-alkinyl, wie 3-Chlorpropargyl.

Hydroxyniederalkyl bedeutet insbesondere Hydroxy-$C_1$-$C_4$-alkyl, wie Hydroxymethyl, 2-Hydroxyethyl oder 3-Hydroxypropyl.

Hydroxyniederalkenyl bedeutet insbesondere Hydroxy-$C_3$-$C_5$-alkenyl, wie 3-Hydroxyallyl.

Hydroxyniederalkinyl bedeutet insbesondere Hydroxy-$C_3$-$C_5$-alkinyl, wie 3-Hydroxypropargyl.

Phenylniederalkyl ist insbesondere Phenyl-$C_1$-$C_4$-alkyl und bedeutet vorzugsweise Benzyl oder 1- oder 2-Phenethyl, während Phenylniederalkenyl bzw. Phenylniederalkinyl insbesondere Phenyl-$C_3$-$C_5$-alkenyl bzw. -alkinyl bedeuten, insbesondere 3-Phenylallyl oder 3-Phenylpropargyl.

Niederalkylen bedeutet insbesondere $C_2$-$C_7$-Alkylen, ist geradkettig oder verzweigt und bedeutet insbesondere Ethylen, 1,3-Propylen, 1,4-Butylen, 1,2-Propylen, 2-Methyl-1,3-propylen oder 2,2-Dimethyl-1,3-propylen. Bevorzugt ist $C_2$-$C_5$-Alkylen.

Niederalkylenoxyniederalkylen bedeutet insbesondere $C_2$-$C_4$-Alkylenoxy-$C_2$-$C_4$-alkylen, vorzugsweise

Ethylenoxyethylen.

Niederalkylamino bedeutet insbesondere $C_1$-$C_7$-Alkylamino und ist z.B. Methyl-, Ethyl-, n-Propyl- oder Isopropyl-amino. Bevorzugt ist $C_1$-$C_4$-Alkylamino.

Niederalkenylamino bedeutet vorzugsweise $C_3$-$C_5$-Alkenylamino, wie Allyl- oder Methallyl-amino.

Niederalkinylamino bedeutet vorzugsweise $C_3$-$C_5$-Alkinylamino, wie Propargylamino.

Phenylniederalkylamino bedeutet vorzugsweise Phenyl-$C_1$-$C_4$-alkylamino, insbesondere Benzyl- oder 1- oder 2-Phenylethyl-amino.

Phenylniederalkenylamino bedeutet vorzugsweise Phenyl-$C_3$-$C_5$-alkenylamino, insbesondere Phenylallylamino oder 3-Phenylmethallylamino.

Phenylniederalkinylamino bedeutet vorzugsweise Phenyl-$C_3$-$C_5$-alkinylamino, insbesondere Phenylpropargylamino.

Diniederalkylamino bedeutet insbesondere Di-$C_1$-$C_4$-alkylamino, wie Dimethyl-, Diethyl-, Di(n-propyl)-, Methyl-propyl-, Methyl-ethyl-, Methyl-butyl- oder Dibutyl-amino.

N-Niederalkyl-N-phenylniederalkyl-amino bedeutet insbesondere N-$C_1$-$C_4$-Alkyl-N-phenyl-$C_1$-$C_4$-alkylamino, vorzugsweise Methyl-benzyl-amino oder Ethyl-benzyl-amino.

Di(phenylniederalkyl)amino bedeutet insbesondere Di(phenyl-$C_1$-$C_4$-alkyl)amino, vorzugsweise Dibenzylamino.

Niederalkenyloxyniederalkyl bedeutet insbesondere $C_3$-$C_5$-Alkenyloxy-$C_1$-$C_4$-alkyl, wie 2-Allyloxyethyl, und Niederalkenyloxy-niederalkenyl bzw. -niederalkinyl bedeutet insbesondere $C_3$-$C_5$-Alkenyloxy-$C_3$-$C_5$-alkenyl bzw. -alkinyl.

Niederalkylthio-niederalkenyl bzw. -niederalkinyl bedeutet insbesondere $C_1$-$C_4$-Alkylthio-$C_3$-$C_5$-alkenyl bzw. -alkinyl.

Niederalkylthioniederalkyl bedeutet insbesondere $C_1$-$C_4$-Alkylthio-$C_1$-$C_4$-alkyl, wie Ethylthiomethyl, 2-Ethylthioethyl, 2-Methylthioethyl oder 2-Isopropylthioethyl, während als Niederalkan-sulfinylniederalkyl bzw. -sulfonylniederalkyl insbesondere entsprechende $C_1$-$C_4$-Alkan-sulfinyl-$C_1$-$C_4$-alkylreste bzw. -sulfonyl-$C_1$-$C_4$-alkylreste in Frage kommen.

Niederalkenylthioniederalkyl ist insbesondere $C_3$-$C_5$-Alkenylthio-$C_1$ -$C_4$-alkyl, wie 1-Allylthioethyl or 3-Allylthiopropyl, waehrend Niederalkenyl-sulfinylniederalkyl bzw. -sulfonylniederalkyl insbesondere $C_3$-$C_5$-Alkenyl-sulfinyl-$C_1$-$C_4$-alkyl bzw. -sulfonyl-$C_1$-$C_4$-alkyl bedeutet.

Niederalkinylthioniederalkyl ist insbesondere $C_3$-$C_5$-Alkinylthio-$C_1$-$C_4$-alkyl, wie 2-Propargylthioethyl oder 3-Propargylthiopropyl, während Niederalkinyl-sulfinylniederalkyl bzw. -sulfonylniederalkyl insbesondere $C_3$-$C_5$-Alkinyl-sulfinyl-$C_1$-$C_4$-alkyl bzw. -sulfonyl-$C_1$-$C_4$-alkyl bedeutet.

Cycloalkyl ist insbesondere $C_3$-$C_7$-Cycloalkyl, d. h. Cyclopropyl, Cyclobutyl, Cyclopentyl, Cyclohexyl oder Cycloheptyl. Bevorzugt sind Cyclopentyl und Cyclohexyl.

Cycloalkenyl ist insbesondere $C_3$-$C_7$-Cycloalkenyl und bedeutet vorzugsweise Cyclopent-2-enyl oder -3-enyl oder Cyclohex-2-enyl oder -3-enyl.

Ausgedehnte pharmakologische Untersuchungen haben ergeben, dass die Verbindungen I und ihre Tautomeren und/oder ihre pharmazeutisch verwendbaren Salze z. B. ausgeprägte Angiotensin-II-antagonisierende Eigenschaften aufweisen.

Bekanntlich hat Angiotensin-II starke vasokonstriktorische Eigenschaften und stimuliert ausserdem die Aldosteronsekretion und bewirkt somit eine deutliche Natrium/Wasser-Retention. Die Folge der Angiotensin-II-Aktivität manifestiert sich unter anderem in einer Erhöhung des Blutdrucks. Die Bedeutung von Angiotensin-II-Antagonisten besteht darin, durch kompetitive Hemmung der Bindung von Angiotensin-II an die Rezeptoren die durch Angiotensin-II bewirkten vasokonstriktorischen und die Aldosteronsekretionstimulierenden Effekte zu unterdrücken.

Die Angiotensin-II-antagonisierenden Eigenschaften der Verbindungen der Formel I und ihrer Tautomeren und/oder ihrer pharmazeutisch verwendbaren Salze können im Angiotensin-II-Bindungstest erfasst werden. Dabei werden glatte Muskelzellen der Ratte aus homogenisierter Rattenaorta verwendet. Das feste Zentrifugat wird in 50 mM Tris-Puffer (pH 7,4) unter Einsatz von Peptidaseinhibitoren suspendiert. Die Proben werden 60 Minuten bei 25 °C mit $^{125}$I-Angiotensin-II (0,175 nM) und einer variierenden Konzentration an Angiotensin-II oder an Testsubstanz inkubiert. Die Inkubation wird dann durch Zugabe von mit eiskaltem Phosphat gepuffertem Kochsalz beendet und es wird durch Whatman GF/F Filter filtriert. Die Filter werden mit einem Gamma-Zähler gezählt. Aus der Dosis-Wirkungs-Kurve werden die $IC_{50}$-Werte bestimmt. Für die Verbindungen der Formel I und ihre pharmazeutisch verwendbaren Salze werden $IC_{50}$-Werte ab etwa 10 nM ermittelt.

Zur Bestimmung der Angiotensin-II induzierten Vasokonstriktion können Untersuchungen an dem isolierten Kaninchen-Aortaring herangezogen werden. Hierzu werden von jeder Brust Aortaringe präpariert und zwischen 2 parallelen Klammern bei einer anfänglich bestehenden Spannung von 2 g fixiert. Anschliessend werden die Ringe bei 37° C in 20 ml eines Gewebebades getaucht und mit einem Gemisch aus 95 % $O_2$ und 5 % $CO_2$ begast. Die isometrischen Reaktionen werden gemessen. In 20-minütigen Intervallen werden die Ringe abwechselnd mit 10 nM Angiotensin-II (Hypertensin-CIBA) und 5 nM Noradrenalinchlorid stimuliert. Anschliessend werden die Ringe mit ausgewählten Konzentrationen der Testsubstanzen vor der Behandlung mit den Agonisten inkubiert. Die Daten werden mit einem Buxco Digitalcomputer analysiert. Die Konzentrationen, die eine 50%-ige Hemmung der Anfangskontrollwerte bewirken, werden als $IC_{50}$-Werte angegeben. Für die Verbindungen der Formel I und ihre Tautomeren und/oder ihre pharmazeutisch verwendbaren Salze werden $IC_{50}$-Werte ab etwa 5 nM bestimmt.

Dass die Verbindungen der Formel I und ihre Tautomeren und/oder ihre pharmazeutisch verwendbaren Salze durch Angiotensin-II induzierten Bluthochdruck reduzieren können, kann im Testmodell der normotensiven, narkotisierten Ratte verifiziert werden. Nach Kalibration der Präparationen mit jeweils 0,9 % NaCl (1 ml/kg i.v.), Noradrenalin (1 µg/kg i.v.) bzw. Angiotensin-II (0,3 µg/kg i.v.) werden steigende Dosen (3-6) der Testsubstanz durch Bolusinjektion intravenös injiziert, worauf nach jeder Dosis in 5 Minuten-Intervallen Angiotensin-II bzw. Noradrenalin appliziert wird. Der Blutdruck wird direkt in der Halsschlagader gemessen und mit einem on-line Datenerfassungssystem aufgezeichnet (Buxco). Die Spezifität des Angiotensin-II-Antagonismus wird angezeigt durch die selektive Hemmung des von Angiotensin-II, nicht aber des durch Noradrenalin hervorgerufenen Druckeffektes. In diesem Testmodell zeigen die Verbindungen der Formel I und ihre Tautomeren und/oder ihre pharmazeutisch verwendbaren Salze ab einer Dosis von etwa 0,3 mg/kg i.v. einen hemmenden Effekt.

Auch im Testmodell der renalen hypertensiven Ratte kann die antihypertensive Aktivität der Verbindungen der Formel I und ihrer Tautomeren und/oder ihrer pharmazeutisch verwendbaren Salze manifestiert werden. Bei männlichen Ratten wird durch Verengung einer renalen Arterie gemäss der Goldblatt-Methode Bluthochdruck erzeugt. Den Ratten werden mittels einer Magensonde Dosen der Testsubstanz verabreicht. Kontrolltiere erhalten ein äquivalentes Volumen an Lösungsmittel. Blutdruck und Herzschlag werden indirekt an wachen Tieren nach der Schwanzklemm-Methode von Gerold et al. [Helv. Physiol. Acta 24 (1966), 58] vor Verabreichung der Testsubstanz bzw. des Lösungsmittels sowie während des Verlaufs der Experimente in Intervallen gemessen. Der ausgeprägte antihypertensive Effekt kann ab einer Dosis von etwa 30 mg/kg p.o. nachgewiesen werden.

Dementsprechend können die Verbindungen der Formel I und ihre Tautomeren und/oder ihre pharmazeutisch verwendbaren Salze z.B. als Wirkstoffe in Antihypertensiva verwendet werden, welche z.B. zur Behandlung von Bluthochdruck sowie von Herzinsuffizienz eingesetzt werden. Ein Erfindungsgegenstand ist somit die Verwendung der Verbindungen der Formel I und ihrer Tautomeren und/oder ihrer pharmazeutisch verwendbaren Salze zur Herstellung von entsprechenden Arzneimitteln und zur therapeutischen Behandlung von Bluthochdruck sowie von Herzinsuffizienz. Bei der Herstellung der Arzneimittel ist auch die gewerbsmässige Herrichtung der Wirksubstanzen eingeschlossen.

Die Erfindung betrifft insbesondere Verbindungen der Formel I, worin einer der Reste $R_1$, $R_2$ und $R_3$ jeweils gegebenenfalls durch Halogen oder Hydroxy substituiertes Niederalkyl, Niederalkenyl oder Niederalkinyl, jeweils 3- bis 7-gliedriges Cycloalkyl oder Cycloalkenyl, Phenylniederalkyl, Phenylniederalkenyl oder Phenylniederalkinyl bedeutet, der zweite der Reste $R_1$, $R_2$ und $R_3$ und der Rest $R_4$ jeweils unabhängig voneinander Halogen, Niederalkanoyl, gegebenenfalls substituiertes Phenyl, Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder disubstituiert oder durch Niederalkylen oder Niederalkylenoxyniederalkylen disubstituiert ist, Cyano, $SO_3H$, $PO_2H_2$, $PO_3H_2$, 5-Tetrazolyl, Sulfamoyl, Niederalkansulfamoyl, Diniederalkansulfamoyl, Niederalkanoylamino, gegebenenfalls substituiertes Benzoylamino, Niederalkansulfonylamino, Halogenniederalkansulfonylamino, gegebenenfalls substituiertes Benzolsulfonylamino oder $-Z_1-R'$, worin $Z_1$ für eine Bindung oder für O, $S(O)_m$ oder N(R) steht, $R'$ Wasserstoff oder jeweils gegebenenfalls durch Hydroxy, Halogen, Amino, Niederalkylenamino, Niederalkylenoxyniederalkylenamino, Niederalkylamino, Niederalkenylamino, Niederalkinylamino, Phenylniederalkylamino, Phenylniederalkenylamino, Phenylniederalkinylamino, Diniederalkylamino, N-Niederalkyl-N-phenylniederalkyl-amino, Di(phenylniederalkyl)amino, Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, oder Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder di-substituiert oder durch Niederalkylen oder Niederalkylenoxyniederalkylen disubstituiert ist, substituiertes Niederalkyl, Niederalkenyl, Niederalkinyl, Niederalkoxyniederalkyl, Niederalkoxyniederalkenyl, Niederalkoxyniederalkinyl, Niederalkenyloxyniederalkyl,

Niederalkenyloxyniederalkenyl, Niederalkenyloxyniederalkinyl, Niederalkylthio-niederalkyl, -niederalkenyl oder -niederalkinyl, Niederalkan-sulfinylniederalkyl oder -sulfonylniederalkyl, Niederalkenyl-thioniederalkyl, -sulfinylniederalkyl oder -sulfonylniederalkyl oder Niederalkinyl-thioniederalkyl, -sulfinylniederalkyl oder -sulfonylniederalkyl bedeutet, R Wasserstoff, Niederalkyl, Niederalkenyl oder Niederalkinyl bedeutet und m für 0, 1 oder 2 steht, bedeuten und der dritte der Reste $R_1$, $R_2$ und $R_3$ für die Gruppe der Formel Ia steht, worin $Z_2$ für Methylen, Niederalkylen, O, $S(O)_m$ oder N(R) steht, $R_5$ Carboxy, Halogenalkansulfonylamino, $SO_3H$, $PO_2H_2$, $PO_3H_2$ oder 5-Tetrazolyl bedeutet, R Wasserstoff, Niederalkyl, Niederalkenyl oder Niederalkinyl bedeutet, m für 0,1 oder 2 steht und die Ringe A und B unabhängig voneinander gegebenenfalls durch Halogen, durch gegebenenfalls durch Hydroxy oder Halogen substituiertes Niederalkyl, Niederalkenyl, Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl oder Niederalkenyloxyniederalkinyl, durch Hydroxy, durch Niederalkoxy, durch Niederalkenyloxy, durch Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, durch Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder di-substituiert oder durch Niederalkylen oder Niederalkylenoxyniederalkylen disubstituiert ist, oder durch 5-Tetrazolyl substituiert sind, wobei die aromatischen Substituenten jeweils gegebenenfalls durch Niederalkyl, Niederalkoxy, Halogen, Trifluormethyl und/oder Hydroxy substituiert sind, und gegebenenfalls Tautomere davon, jeweils in freier Form oder in Salzform.

Die Erfindung betrifft insbesondere Verbindungen der Formel I, worin $R_1$ Halogen, Carboxy, welches gegebenenfalls durch einen Alkohol verestert ist, der sich von Niederalkyl oder Niederalkoxyniederalkyl ableitet, Carbamoyl, Cyano, $PO_3H_2$, 5-Tetrazolyl, Niederalkansulfamoyl, Niederalkanoylamino, Niederalkansulfonylamino oder $-Z_1-R'$, worin $Z_1$ für eine Bindung oder für O, $S(O)_m$ oder N(R) steht, $R'$ Wasserstoff oder jeweils gegebenenfalls durch Carboxy, Niederalkoxycarbonyl, Niederalkoxynieder alkoxycarbonyl oder Hydroxy substituiertes Niederalkyl oder Niederalkoxyniederalkyl bedeutet, R Wasserstoff oder Niederalkyl ist und m für 0,1 oder 2 steht, bedeutet, $R_2$ für die Gruppe der Formel Ia steht, worin $Z_2$ für Methylen, Niederalkylen, O, $S(O)_m$ oder N(R) steht, R Wasserstoff oder Niederalkyl bedeutet, m für 0,1 oder 2 steht, $R_5$ Carboxy oder 5-Tetrazolyl bedeutet und die Ringe A und B unabhängig voneinander gegebenenfalls durch Halogen, Niederalkyl, Niederalkoxy, Carboxy, Niederalkoxycarbonyl oder 5-Tetrazolyl substituiert sind, $R_3$ gegebenenfalls durch Hydroxy oder Halogen substituiertes Niederalkyl oder Niederalkenyl bedeutet und $R_4$ Niederalkyl bedeutet, und gegebenenfalls Tautomere davon, jeweils in freier Form oder in Salzform.

Die Erfindung betrifft insbesondere Verbindungen der Formel I, worin $R_1$ Halogen, insbesondere mit einer Atomnummer bis und mit 35, wie Chlor, Carboxy, Niederalkoxycarbonyl, wie Methoxy- oder Ethoxycarbonyl, $PO_3H_2$, 5-Tetrazolyl, Niederalkanoylamino, wie Acetylamino, Niederalkansulfonylamino, wie Methansulfonylamino, Wasserstoff, Niederalkyl, wie Methyl, Niederalkoxyniederalkyl, wie 2-Methoxyethyl, Carboxyniederalkyl, wie Carboxymethyl, Niederalkoxycarbonylniederalkyl, wie Ethoxycarbonylmethyl, Hydroxyniederalkyl, wie Hydroxymethyl, Hydroxyniederalkoxyniederalkyl, wie Hydroxyethoxymethyl, Hydroxy, Niederalkoxy, wie Methoxy, Niederalkoxyniederalkoxy, wie 2-Methoxyethoxy, Carboxyniederalkoxy, wie Carboxymethoxy, Niederalkoxycarbonylniederalkoxy, wie Ethoxycarbonylmethoxy, Hydroxyniederalkoxy, wie 2-Hydroxyethoxy, Mercapto, Niederalkylthio, wie Methylthio, Niederalkansulfinyl, wie Methansulfinyl, Niederalkansulfonyl, wie Methansulfonyl, Amino, Niederalkylamino, wie Methylamino, oder Diniederalkylamino, wie Dimethylamino, bedeutet, $R_2$ für die Gruppe der Formel Ia steht, worin $Z_2$ für Methylen oder Niederalkylen, wie Ethylen, O oder $S(O)_m$ steht, m für 0,1 oder 2 steht, $R_5$ Carboxy oder 5-Tetrazolyl bedeutet und die Ringe A und B unabhängig voneinander gegebenenfalls durch Niederalkyl, wie Methyl, Halogen, insbesondere mit einer Atomnummer bis und mit 35, wie Chlor, oder Niederalkoxy, wie Methoxy, substituiert sind, $R_3$ $C_1$-$C_7$-Alkyl, wie n-Propyl oder n-Butyl, bedeutet und $R_4$ Niederalkyl, wie Methyl oder n-Butyl, bedeutet, wobei mit "nieder" bezeichnete Teilstrukturen jeweils insbesondere bis und mit 7, vorzugsweise bis und mit 4, C-Atome umfassen, und gegebenenfalls Tautomere davon, jeweils in freier Form oder in Salzform.

Die Erfindung betrifft in erster Linie Verbindungen der Formel I, worin $R_2$ für die Gruppe der Formel

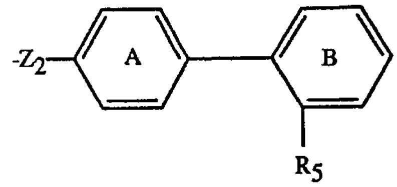

(Ib)

steht, und gegebenenfalls Tautomere davon, jeweils in freier Form oder in Salzform.

Die Erfindung betrifft in erster Linie Verbindungen der Formel I, worin $R_1$ Halogen mit einer Atomnummer bis und mit 35, wie Chlor, Hydroxy, Niederalkoxy mit bis und mit 4 C-Atomen, wie Methoxy, Niederalkoxyniederalkoxy mit bis und mit 4 C-Atomen in jedem Niederalkoxyteil, wie 2-Methoxyethoxy, Hydroxyniederalkoxy mit bis und mit 4 C-Atomen, wie 2-Hydroxyethoxy, Niederalkylamino mit bis und mit 4 C-Atomen, wie Methylamino, oder Diniederalkylamino mit bis und mit 4 C-Atomen in jedem Niederalkylteil, wie Dimethylamino, bedeutet, $R_2$ für die Gruppe der Formel Ib steht, worin $Z_2$ Methylen bedeutet, $R_5$ Carboxy oder insbesondere 5-Tetrazolyl bedeutet und die Ringe A und B unsubstituiert sind, $R_3$ $C_1$-$C_7$-Alkyl, insbesondere $C_1$-$C_5$-Alkyl, wie n-Propyl oder n-Butyl, bedeutet und $R_4$ Niederalkyl mit bis und mit 4 C-Atomen, wie Methyl oder n-Butyl, bedeutet, und gegebenenfalls Tautomere davon, jeweils in freier Form oder in Salzform.

Die Erfindung betrifft in erster Linie Verbindungen der Formel I, worin $R_1$ Hydroxy bedeutet, $R_2$ für die Gruppe der Formel Ib steht, worin $Z_2$ Methylen bedeutet, $R_5$ 5-Tetrazolyl bedeutet und die Ringe A und B unsubstituiert sind, $R_3$ $C_1$-$C_5$-Alkyl, wie n-Propyl oder n-Butyl, bedeutet und $R_4$ Niederalkyl mit bis und mit 4 C-Atomen, wie Methyl oder n-Butyl, bedeutet, und gegebenenfalls Tautomere davon, jeweils in freier Form oder in Salzform.

Die Erfindung betrifft namentlich die in den Beispielen genannten neuen Verbindungen der Formel I und gegebenenfalls deren Tautomere, jeweils in freier Form oder in Salzform.

Ein weiterer Gegenstand der Erfindung ist ein Verfahren zur Herstellung der Verbindungen der Formel I und gegebenenfalls ihrer Tautomeren, jeweils in freier Form oder in Salzform, z.B. dadurch gekennzeichnet, dass man

a) in einer Verbindung der Formel

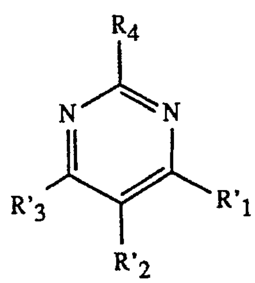

(IIa)

oder einem Tautomeren und/oder Salz davon, worin einer der Reste $R'_1$, $R'_2$ und $R'_3$ für die Gruppe der Formel

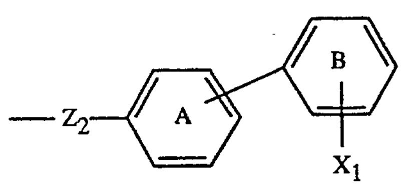

(IIb)

steht, worin $X_1$ ein in $R_5$ überführbaren Rest ist, und die beiden anderen Reste die von der Gruppe der Formel Ia verschiedenen Bedeutungen von $R_1$, $R_2$ bzw. $R_3$ haben, $X_1$ in $R_5$ überführt oder

b) eine Verbindung der Formel

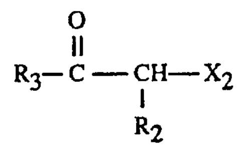

(IIIa),

worin $X_2$ gegebenenfalls funktionell abgewandeltes Carboxy bedeutet, mit einer Verbindung der Formel

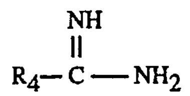

(IIIb)

oder einem Salz davon umsetzt und jeweils, wenn erwünscht, eine verfahrensgemäss oder auf andere Weise erhältliche Verbindung der Formel I oder ein Tautomeres davon, jeweils in freier Form oder in Salzform, in eine andere Verbindung der Formel I oder ein Tautomeres davon überführt, ein verfahrensgemäss erhältliches Gemisch von Isomeren auftrennt und das gewünschte Isomere isoliert und/oder eine verfahrensgemäss erhältliche freie Verbindung der Formel I oder ein Tautomeres davon in ein Salz oder ein verfahrensgemäss erhältliches Salz einer Verbindung der Formel I oder eines Tautomeren davon in die freie Verbindung der Formel I oder ein Tautomeres davon oder in ein anderes Salz überführt.

Für Tautomere bzw. Salze von Ausgangsmaterialien gilt das vorstehend für Tautomere bzw. Salze von Verbindungen I Gesagte in analoger Weise.

In die Variable $R_5$ überführbare Reste $X_1$ sind beispielsweise Cyano, Mercapto, Halogen, die Gruppe $-N_2^+A^-$, in der $A^-$ ein von einer Säure abgeleitetes Anion bedeutet, Amino, funktionelle Derivate von COOH, $SO_3H$, $PO_3H_2$ und $PO_2H_2$ und N-geschütztes 5-Tetrazolyl.

Funktionell abgewandeltes Carboxy bedeutet beispielsweise Cyano oder verestertes oder amidiertes Carboxy.

Die vor- und nachstehend in den Varianten beschriebenen Umsetzungen werden in an sich bekannter Weise durchgeführt, z.B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -80° C bis zur Siedetemperatur des Reaktionsmediums, vorzugsweise von etwa -10° bis etwa +200° C, und, falls erforderlich, in einem geschlossenen Gefäss, unter Druck, in einer Inertgasatmosphäre und/oder unter wasserfreien Bedingungen arbeitet.

<u>Variante a):</u>

In 5-Tetrazolyl $R_5$ überführbare Reste $X_1$ sind beispielsweise Cyano und N-geschütztes 5-Tetrazolyl.

Zur Herstellung von Verbindungen der Formel I, worin $R_5$ 5-Tetrazolyl bedeutet, geht man beispielsweise von Ausgangsmaterial der Formel IIa aus, worin $X_1$ Cyano bedeutet, und setzt dieses mit einem Azid, z. B. mit $HN_3$ oder insbesondere einem Salz, wie Alkalimetallsalz, davon oder mit einem Organozinnazid, wie Triniederalkyl- oder Triarylzinnazid, um. Bevorzugte Azide sind beispielsweise Natrium- und Kaliumazid sowie Tri-$C_1$-$C_4$-alkyl-, z.B. Triethyl- oder Tributyl-zinnazid, und Triphenylzinnazid.

Als Schutzgruppen von N-geschütztem 5-Tetrazolyl kommen die üblicherweise in der Tetrazolchemie verwendeten Schutzgruppen in Frage, insbesondere Triphenylmethyl, gegebenenfalls, z.B. durch Nitro, substituiertes Benzyl, wie 4-Nitrobenzyl, Niederalkoxymethyl, wie Methoxy- oder Ethoxymethyl, Niederalkylthiomethyl, wie Methylthiomethyl, sowie 2-Cyanoethyl, ferner Niederalkoxyniederalkoxymethyl, wie 2-Methoxyethoxymethyl, Benzyloxymethyl sowie Phenacyl. Die Abspaltung der Schutzgruppen erfolgt in Anlehnung an bekannte Methoden. So wird z.B. Triphenylmethyl üblicherweise durch Hydrolyse, insbesondere in Gegenwart einer Säure, oder Hydrogenolyse in Gegenwart eines Hydrierungskatalysators, 4-Nitrobenzyl z.B. durch Hydrogenolyse in Gegenwart eines Hydrierungskatalysators, Methoxy- oder Ethoxymethyl z.B. durch Behandeln mit einem Triniederalkyl-, wie Triethyl- oder Tributyl-zinn-bromid, Methylthiomethyl z.B. durch Behandeln mit Trifluoressigsäure, 2-Cyanoethyl z.B. durch Hydrolyse, beispielsweise mit Natronlauge, 2-Methoxyethoxymethyl z.B. durch Hydrolyse, z.B. mit Salzsäure, und Benzyloxymethyl und Phenacyl z.B. durch Hydrogenolyse in Gegenwart eines Hydrierungskatalysators abgespalten.

Ein in $SO_3H$ $R_5$ überführbarer Rest $X_1$ ist beispielsweise die Mercaptogruppe. Eine solche Gruppe aufweisende Ausgangsverbindungen der Formel IIa werden beispielsweise durch an sich bekannte Oxidationsverfahren zu solchen Verbindungen der Formel I oxidiert, worin $R_5$ $SO_3H$ ist. Als Oxidationsmittel kommen beispielsweise anorganische Persäuren, wie Persäuren von Mineralsäuren, z.B. Periodsäure oder Perschwefelsäure, organische Persäuren, wie Percarbon- oder Persulfon-säuren, z.B. Perameisen-, Peressig-, Trifluorperessig- oder Perbenzoe-säure oder p-Toluolpersulfonsäure, oder Gemische aus Wasserstoffperoxid und Säuren, z.B. Gemische aus Wasserstoffperoxid und Essigsäure, in Betracht. Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Übergangsmetalloxide, wie Oxide von Elementen der VI. Nebengruppe, z.B. Molybdän- oder Wolframoxid, zu nennen sind. Die Oxidation wird unter milden Bedingungen, z.B. bei Temperaturen von etwa -50° bis etwa +100° C, durchgeführt.

Unter einer in $PO_3H_2$ $R_5$ überführbaren Gruppe ist beispielsweise eine Gruppe $-N_2^+$ $A^-$ zu verstehen, wobei $A^-$ für ein Anion einer Säure, wie Mineralsäure, steht. Entsprechende Diazoniumverbindungen werden beispielsweise in an sich bekannter Weise mit einem P(III)-Halogenid, wie $PCl_3$ oder $PBr_3$, umgesetzt und hydrolytisch aufgearbeitet, wobei solche Verbindungen der Formel I erhältlich sind, worin $R_5$ $PO_3H_2$ ist.

Verbindungen I, worin $R_5$ $PO_2H_2$ ist, erhält man z. B. durch in üblicher Weise erfolgende Umwandlung von $X_1$ in einer Verbindung IIa, worin $X_1$ ein funktionelles Derivat von $PO_2H_2$ ist, in $PO_2H_2$.

Als in Halogenalkansulfonylamino $R_5$ überführbarer Rest $X_1$ kommt beispielsweise Amino in Frage. Zur Herstellung von Verbindungen der Formel I, worin $R_5$ Halogenalkansulfonylamino bedeutet, setzt man beispielsweise entsprechende Aniline mit einer üblicherweise reaktionsfähig veresterten Halogenalkansulfonsäure um, wobei gegebenenfalls in Gegenwart einer Base gearbeitet wird. Die bevorzugte reaktionsfähig veresterte Halogenalkansulfonsäure ist das entsprechende Halogenid, wie Chlorid oder Bromid.

Ein in COOH $R_5$ überführbarer Rest $X_1$ steht beispielsweise für funktionell abgewandeltes Carboxy, wie Cyano, verestertes oder amidiertes Carboxy, Hydroxymethyl oder Formyl.

Verestertes Carboxy ist beispielsweise mit einem gegebenenfalls substituierten aliphatischen, cycloaliphatischen oder aromatischen Alkohol verestertes Carboxy. Ein aliphatischer Alkohol ist beispielsweise ein Niederalkanol, wie Methanol, Ethanol, n-Propanol, Isopropanol, n-Butanol, sek.-Butanol oder tert.-Butanol, während als cycloaliphatischer Alkohol beispielsweise ein 3- bis 8-gliedriges Cycloalkanol, wie Cyclopentanol, -hexanol oder -heptanol, in Frage kommt. Ein aromatischer Alkohol ist beispielsweise ein Phenol oder ein heterocyclischer Alkohol, welche jeweils substituiert sein können, insbesondere Hydroxypyridin, z.B. 2-, 3- oder 4-Hydroxypyridin.

Amidiertes Carboxy ist beispielsweise Carbamoyl, durch Hydroxy, Amino oder gegebenenfalls substituiertes Phenyl monosubstituiertes, durch Niederalkyl mono- oder disubstituiertes oder durch 4- bis 7-gliedriges Alkylen oder 3-Aza-, 3-Niederalkylaza-, 3-Oxa- oder 3-Thiaalkylen disubstituiertes Carbamoyl. Als Beispiele sind Carbamoyl, N-Mono- oder N,N-Diniederalkylcarbamoyl, wie N-Methyl-, N-Ethyl-, N,N-Dimethyl-, N,N-Diethyl- und N,N-Dipropyl-carbamoyl, Pyrrolidino- und Piperidinocarbonyl, Morpholino-, Piperazino-, 4-Methylpiperazino- und Thiomorpholino-carbonyl, Anilinocarbonyl und durch Niederalkyl, Niederalkoxy und/oder Halogen substituiertes Anilinocarbonyl zu nennen.

Bevorzugtes funktionell abgewandeltes Carboxy ist beispielsweise Niederalkoxycarbonyl, wie Methoxy- oder Ethoxycarbonyl, und Cyano.

Verbindungen der Formel I, worin $R_5$ Carboxy ist, können beispielsweise ausgehend von Verbindungen der Formel IIa, worin $X_1$ Cyano oder verestertes oder amidiertes Carboxy bedeutet, durch Hydrolyse, insbesondere in Gegenwart einer Base, oder, ausgehend von Verbindungen der Formel IIa, worin $X_1$ Hydroxymethyl oder Formyl bedeutet, durch Oxidation hergestellt werden. Die Oxidation erfolgt beispielsweise in einem inerten Lösungsmittel, wie in einer Niederalkancarbonsäure, z.B. Essigsäure, einem Keton, z.B. Aceton, einem Ether, z.B. Tetrahydrofuran, einem heterocyclischen Aromaten, z.B. Pyridin, oder in Wasser, oder in einem Gemisch davon, erforderlichenfalls unter Kühlen oder Erwärmen, z.B. in einem Temperaturbereich von etwa 0° bis etwa +150° C. Als Oxidationsmittel kommen beispielsweise oxidierende Übergangsmetallverbindungen, insbesondere solche mit Elementen der I., VI. oder VII. Nebengruppe, in Frage. Als Beispiele seien genannt: Silberverbindungen, wie Silber-nitrat, -oxid und -picolinat, Chromverbindungen, wie Chromtrioxid und Kaliumdichromat, und Manganverbindungen, wie Kalium-, Tetrabutylammonium- und Benzyltriethylammonium-permanganat. Weitere Oxidationsmittel sind beispielsweise geeignete Verbindungen mit Elementen der IV. Hauptgruppe, wie Bleidioxid, oder Halogen-Sauerstoff-Verbindungen, wie Natriumiodat oder Kaliumperiodat.

Vorzugsweise eignet sich die Variante a) zur Herstellung solcher Verbindungen der Formel I, worin die Variablen Bedeutungen haben, die von ungesättigten Resten verschieden sind.

Das Ausgangsmaterial der Formel IIa ist beispielsweise zugänglich, indem man in Analogie zu Variante b) von einer Verbindung der Formel

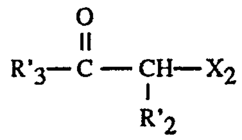

(IIc)

ausgeht, deren Herstellung wiederum in an sich bekannter Weise erfolgt, und diese mit einer Verbindung der Formel IIIb umsetzt. Es ist möglich, in den so erhaltenen Ausgangsverbindungen IIa vor der Umwandlung von $X_1$ in $R_5$ noch weitere Umwandlungen vorzunehmen, z. B. Umwandlungen eines Halogenatoms $R_1$, insbesondere eines Chloratoms $R_1$, der nachstehend angegebenen Art.

Variante b):

Die Umsetzung wird erforderlichenfalls in Gegenwart einer Base durchgeführt.

Als Basen kommen beispielsweise Alkalimetall-hydroxide, -hydride, -amide, -alkanolate, -carbonate, -triphenylmethylide, -diniederalkylamide, -aminoalkylamide oder -niederalkylsilylamide, Naphthalinamine, Niederalkylamine, basische Heterocyclen, Ammoniumhydroxide, sowie carbocyclische Amine in Frage. Beispielhaft seien Natriumhydroxid, -hydrid, -amid, Kalium-tert-butylat, -carbonat, Lithium-triphenylmethylid, -diisopropylamid, Kalium-3-(aminopropyl)-amid, -bis-(trimethylsilyl)-amid, Dimethylaminonaphthalin, Di- oder Triethylamin, oder Ethyl-diisopropylamin, N-Methyl-piperidin, Pyridin, Benzyltrimethyl-ammoniumhydroxid, 1,5-Diazabicyclo[4.3.0]non-5-en (DBN) sowie 1,8-Diaza-bicyclo[5.4.0]undec-7-en (DBU) genannt.

Die Herstellung von Verbindungen der Formel IIa kann z.B. analog der vorstehend beschriebenen Herstellungsweise von Verbindungen der Formel IIc erfolgen. Verbindungen der Formel IIIb sind bekannt oder können unter Verwendung an sich bekannter Methoden hergestellt werden.

Eine verfahrensgemäss oder auf andere Weise erhältliche Verbindung der Formel I oder ein Tautomeres davon kann in an sich bekannter Weise in eine andere Verbindung der Formel I überführt werden.

Eine Hydroxy aufweisende Verbindung der Formel I kann z. B. nach an sich bekannten Methoden verethert werden. Die Veretherung kann z.B. mit einem Alkohol, wie einem gegebenenfalls substituierten Niederalkanol, oder einem reaktionsfähigen Ester desselben erfolgen. Als reaktionsfähige Ester der gewünschten Alkohole kommen beispielsweise solche mit starken anorganischen oder organischen Säuren in Frage, wie entsprechende Halogenide, Sulfate, Niederalkansulfonate oder gegebenenfalls substituierte Benzolsulfonate, z.B. Chloride, Bromide, Iodide oder Methan-, Benzol- oder p-Toluol-sulfonate, in Betracht. Die Veretherung kann z.B. in Gegenwart einer Base, z. B. eines Alkalimetall-hydrids, -hydroxids oder -carbonats oder eines basischen Amins, erfolgen. Umgekehrt können entsprechende Ether, wie Niederalkoxyverbindungen, z.B. mittels starker Säuren, wie Mineralsäuren, z.B. Brom- oder Iod-wasserstoffsäure, die vorteilhaft in Form von Pyridiniumhalogeniden vorliegen können, oder mittels Lewissäuren, z.B. Halogeniden von Elementen der III. Hauptgruppe oder der entsprechenden Nebengruppen, gespalten werden. Diese Umsetzungen können, falls erforderlich, unter Kühlen oder Erwärmen, z.B. in einem Temperaturbereich von etwa -20° bis etwa +100° C, in An- oder Abwesenheit eines Lösungs- oder Verdünnungsmittels, unter Inertgas und/oder unter Druck und gegebenenfalls in einem geschlossenen Gefäss durchgeführt werden.

Falls ein aromatischer Strukturbestandteil durch Niederalkylthio substituiert ist, kann man dieses auf übliche Weise zu entsprechendem Niederalkan-sulfinyl oder -sulfonyl oxidieren. Als geeignetes Oxidationsmittel für die Oxidation zur Sulfoxidstufe kommen beispielsweise anorganische Persäuren, wie Persäuren von Mineralsäuren, z.B. Periodsäure oder Perschwefelsäure, organische Persäuren, wie Percarbon- oder Persulfonsäuren, z.B. Perameisen-, Peressig-, Trifluorperessig-, Perbenzoe- oder p-Toluolpersulfon-säure, oder Gemische aus Wasserstoffperoxid und Säuren, z.B. Gemische aus Wasserstoffperoxid und Essigsäure, in Betracht. Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Übergangsmetalloxide, wie Oxide von Elementen der VI. Nebengruppe, z.B. Molybdän- oder Wolfram-oxid, zu nennen sind. Die Oxidation wird unter milden Bedingungen, z.B. bei Temperaturen von etwa -50° bis etwa +100° C, durchgeführt. Die weitere Oxidation zur Sulfonstufe kann man mit Distickstofftetroxid als Katalysator in Gegenwart von Sauerstoff bei tiefen Temperaturen entsprechend durchführen, ebenso wie die direkte Oxidation von Niederalkylthio zu Niederalkansulfonyl. Jedoch setzt man hierbei üblicherweise das Oxidationsmittel im Überschuss ein.

Weist eine der Variablen Amino auf, können entsprechende Verbindungen I in an sich bekannter Weise N-(ar)alkyliert werden; ebenso können Carbamoyl bzw. Carbamoyl aufweisende Reste N-(ar)alkyliert werden. Die (Ar-)Alkylierung erfolgt z.B. mit einem (Aryl-)$C_1$-$C_7$-Alkyl-halogenid, z.B. -bromid oder -iodid, (Aryl-

)$C_1$-$C_7$-Alkansulfonat, z.B. mit Methansulfonat oder p-Toluolsulfonat, oder einem Di-$C_1$-$C_7$-alkylsulfat, z.B. Dimethylsulfat, vorzugsweise unter basischen Bedingungen, wie in Gegenwart von Natronlauge oder Kalilauge, und vorteilhaft in Gegenwart eines Phasentransfer-Katalysators, wie von Tetrabutylammoniumbromid oder Benzyltrimethylammoniumchlorid, wobei indes stärker basische Kondensationsmittel, wie Alkalimetall-amide, -hydride oder -alkoholate, z.B. Natriumamid, Natriumhydrid oder Natriumethanolat, erforderlich sein können.

In Verbindungen der Formel I, die als Substituenten eine veresterte oder amidierte Carboxygruppe aufweisen, kann man eine solche Gruppe, z.B. mittels Hydrolyse, z.B. in Gegenwart eines basischen Mittels oder eines sauren Mittels, wie einer Mineralsäure, in eine freie Carboxygruppe überführen.

Ferner kann man in Verbindungen der Formel I, die als Substituenten eine Carboxygruppe aufweisen (insbesondere, sofern $R_5$ von Carboxy verschieden ist), diese, z.B. durch Behandeln mit einem Alkohol, wie einem Niederalkanol, in Gegenwart eines geeigneten Veresterungsmittels, wie eines sauren Reagens, z.B. einer anorganischen oder organischen Säure oder einer Lewissäure, z.B. von Zinkchlorid, oder eines wasserbindenden Kondensationsmittels, z.B. eines Carbodiimids, wie von N,N′-Dicyclohexylcarbodiimid, oder durch Behandeln mit einem Diazoreagens, wie mit einem Diazoniederalkan, z.B. Diazomethan, in eine veresterte Carboxygruppe überführen. Diese kann man auch erhalten, wenn man Verbindungen der Formel I, worin die Carboxygruppe in freier Form oder in Salz-, wie Ammonium- oder Metall-, z.B. Alkalimetall-, wie Natrium- oder Kalium-salzform vorliegt, mit einem $C_1$-$C_7$-Alkylhalogenid, z.B. Methyl- oder Ethyl-bromid oder -iodid, oder einem organischen Sulfonsäureester, wie einem entsprechenden $C_1$-$C_7$-Alkylester, z.B. Methansulfonsäure- oder p-Toluolsulfonsäure-methylester oder -ethylester, behandelt.

Verbindungen der Formel I, die als Substituenten eine veresterte Carboxygruppe aufweisen, kann man durch Umesterung, z.B. durch Behandeln mit einem Alkohol, üblicherweise mit einem höheren als dem der veresterten Carboxygruppe im Ausgangsmaterial entsprechenden Alkohol, in Gegenwart eines geeigneten Umesterungsmittels, wie eines basischen Mittels, z.B. eines Alkalimetall-$C_1$-$C_7$-alkanoats, -$C_1$-$C_7$-alkanolats oder -cyanids, wie von Natrium-acetat, -methanolat, -ethanolat, -tert.-butanolat oder -cyanid, oder eines geeigneten sauren Mittels, gegebenenfalls unter Entfernung des entstehenden Alkohols, z.B. durch Destillation, in andere Esterverbindungen der Formel I umwandeln. Man kann auch von entsprechenden, sogenannten aktivierten Estern der Formel I ausgehen, die als Substituenten eine aktivierte veresterte Carboxygruppe aufweisen (siehe unten), und diese durch Behandeln mit einem $C_1$-$C_7$-Alkanol in einen anderen Ester umwandeln.

Man kann in Verbindungen der Formel I, die als Substituenten die Carboxygruppe enthalten, diese auch zuerst in ein reaktionsfähiges Derivat, wie ein Anhydrid (auch ein gemischtes Anhydrid), ein Säurehalogenid, z.B. -chlorid (z.B. durch Behandeln mit einem Thionyl-halogenid, z.B. -chlorid), ein Anhydrid mit einem Ameisensäure-ester, z.B. -$C_1$-$C_7$-alkylester (z.B. durch Behandeln eines Salzes, wie eines Ammonium- oder Alkalimetallsalzes, mit einem Halogen-, wie Chlor-ameisensäureester, wie $C_1$-$C_7$-Alkylester), oder einen aktivierten Ester, wie Cyanmethyl-, Nitrophenyl-, z.B. 4-Nitro-phenyl-, oder Polyhalogenphenyl-, z.B. Pentachlorphenyl-ester (z.B. durch Behandeln mit einer entsprechenden Hydroxyverbindung in Gegenwart eines geeigneten Kondensationsmittels, wie von N,N′-Dicyclohexylcarbodiimid) überführen, und ein solches reaktionsfähiges Derivat dann mit einem Amin umsetzen und so zu Amidverbindungen der Formel I gelangen, die als Substituenten eine amidierte Carboxygruppe aufweisen. Dabei kann man diese direkt oder über Zwischenverbindungen erhalten; so kann man z.B. einen aktivierten Ester, wie einen 4-Nitrophenylester, einer Verbindung der Formel I mit einer Carboxygruppe zuerst mit einem 1-unsubstituierten Imidazol umsetzen und die so entstandene 1-Imidazolylcarbonylverbindung mit einem Amin in Reaktion bringen. Man kann aber auch andere, nicht-aktivierte Ester, wie $C_1$-$C_7$-Alkylester, von Verbindungen der Formel I mit Aminen zur Reaktion bringen.

Weist ein aromatischer Ring als Substituenten ein Wasserstoffatom auf, so kann dieses mit Hilfe eines Halogenierungsmittels in üblicher Weise durch ein Halogenatom ersetzt werden, z.B. mit Brom, Hypobromsäure, einem Acylhypobromit oder einer anderen organischen Bromverbindung, z.B. N-Bromsuccinimid, N-Bromacetamid, N-Bromphthalimid, Pyridiniumperbromid, Dioxandibromid, 1,3-Dibrom-5,5-dimethylhydantoin oder 2,4,4,6-Tetrabrom-2,5-cyclohexandien-1-on, durch Brom oder mit elementarem Chlor, z.B. in einem halogenierten Kohlenwasserstoff, wie Chloroform, und unter Kühlen, z.B. bis auf etwa -10 °C, durch Chlor.

Enthält ein aromatischer Ring eine Aminogruppe, so kann diese in üblicher Weise diazotiert werden, z.B. durch Behandeln mit einem Nitrit, z.B. Natriumnitrit, in Gegenwart einer geeigneten Protonsäure, z.B. einer Mineralsäure, wobei die Reaktionstemperatur vorteilhaft unter etwa 5 °C gehalten wird. Die so erhältliche, in Salzform vorliegende Diazoniumgruppe kann man nach üblichen Verfahren beispielsweise wie folgt substituieren: durch die Hydroxygruppe analog der Phenolverkochung in Gegenwart von Wasser; durch eine Alkoxygruppe durch Behandeln mit einem entsprechenden Alkohol, wobei Energie zugeführt werden muss; durch das Fluoratom analog der Schiemann-Reaktion bei der Thermolyse von entsprechenden Diazoniumtetrafluorboraten; oder durch Chlor, Brom, Iod oder die Cyanogruppe analog der Sandmeyer-Reaktion durch Umsetzung mit entsprechenden Cu(I)-Salzen, zunächst unter Kühlen, z.B. auf unter etwa 5° C, und anschliessendem Erhitzen, z.B. auf etwa 60° bis etwa 150° C.

Enthalten die Verbindungen der Formel I ungesättigte Reste, wie Niederalkenyl oder Niederalkinylgruppierungen, können diese in an sich bekannter Weise in gesättigte Reste überführt werden. So erfolgt beispielsweise die Hydrierung von Mehrfachbindungen durch katalytische Hydrierung in Gegenwart von Hydrierungskatalysatoren, wobei hierfür z.B. Nickel, wie Raney-Nickel, sowie Edelmetalle oder deren Derivate, z.B. Oxide, geeignet sind, wie Palladium oder Platinoxid, die gegebenenfalls auf Trägermaterialien, z.B. auf Kohle oder Calciumcarbonat, aufgezogen sein können. Die Hydrierung kann vorzugsweise bei Drucken zwischen etwa 1 und etwa 100 at und bei Temperaturen zwischen etwa -80° und etwa +200° C, vor allem zwischen Raumtemperatur und etwa 100° C, durchgeführt werden. Die Reaktion erfolgt zweckmässig in einem Lösungsmittel, wie Wasser, einem Niederalkanol, z.B. Ethanol, Isopropanol oder n-Butanol, einem Ether, z.B. Dioxan, oder einer Niederalkancarbonsäure, z.B. Essigsäure.

Weiterhin kann in Verbindungen I, worin z.B. einer der Reste $R_1$, $R_2$, $R_3$ und $R_4$ Halogen, wie Chlor, bedeutet, Halogen durch Umsetzung mit einem gegebenenfalls substituierten Amin, einem Alkohol oder Mercaptan ausgetauscht werden. Ein Halogenatom $R_1$, insbesondere ein Chloratom $R_1$, kann auch durch Umsetzung mit einem Reagens der Formel $Alk_3SnR_6$ (Alk = Niederalkyl; $R_6$ = gegebenenfalls substituiertes Niederalk-1-en-1-yl oder gegebenenfalls substituiertes Niederalk-1-in-1-yl), z. B. in Gegenwart eines Metallkatalysators, wie in Gegenwart von $(P(C_6H_5)_3)_2PdCl_2$, gegen entsprechende Niederalk-1-en-1-yl- oder Niederalk-1-in-1-yl-reste ausgetauscht werden, welche dann weiteren Reaktionen zugänglich sind, z. B. der Ozonolyse oder der Reduktion, welche z. B. zur Bildung der Aldehydfunktion, welche ihrerseits zum Beispiel in Carboxy überführt werden kann, oder zur Bildung einer Niederalkylgruppe führen können. Auch kann ein entsprechendes Chloratom $R_1$, z. B. durch Umsetzung mit CsF, gegen Fluor, welches einer weiteren nucleophilen Substitution, z. B. durch Cyano, leicht zugänglich ist, ausgetauscht werden.

Die Erfindung betrifft insbesondere die in den Beispielen beschriebenen Verfahren.

Salze von Verbindungen der Formel I können in an sich bekannter Weise hergestellt werden. So erhält man beispielsweise Säureadditionssalze von Verbindungen der Formel I durch Behandeln mit einer geeigneten Säure oder einem geeigneten Ionenaus tauscherreagens. Salze von Verbindungen I können in üblicher Weise in die freien Verbindungen I überführt werden, Säureadditionssalze z.B. durch Behandeln mit einem geeigneten basischen Mittel oder einem geeigneten Ionenaustauscherreagens.

Salze von Verbindungen I können in an sich bekannter Weise in andere Salze von Verbindungen I umgewandelt werden.

Je nach Verfahrensweise bzw. Reaktionsbedingungen können die Verbindungen I mit salzbildenden, insbesondere basischen Eigenschaften, in freier Form oder in Form von Salzen erhalten werden.

Infolge der engen Beziehung zwischen der Verbindung I in freier Form und in Form ihrer Salze sind im Vorausgegangenen und nachfolgend unter der freien Verbindung I bzw. ihren Salzen sinn- und zweckgemäss gegebenenfalls auch die entsprechenden Salze bzw. die freie Verbindung I zu verstehen.

Die Verbindungen I einschliesslich ihrer Salze von salzbildenden Verbindungen können auch in Form ihrer Hydrate erhalten werden und/oder andere, z. B. zur Kristallisation verwendete, Lösungsmittel einschliessen.

Die Verbindungen I und ihre Salze können, je nach der Wahl der Ausgangsstoffe und Arbeitsweisen, in Form eines der möglichen Isomeren oder als Gemisch derselben, z.B. je nach Anzahl, absoluter und relativer Konfiguration der asymmetrischen Kohlenstoffatome als reine Isomere, wie Antipoden und/oder Diastereomere, oder als Isomerengemische, wie Enantiomerengemische, z. B. Racemate, Diastereomerengemische oder Racematgemische, vorliegen.

Erhaltene Diastereomerengemische und Racematgemische können auf Grund der physikalisch-chemischen Unterschiede der Bestandteile in bekannter Weise in die reinen Diastereomeren oder Racemate aufgetrennt werden, beispielsweise durch fraktionierte Kristallisation. Erhaltene Enantiomerengemische, wie Racemate, lassen sich nach bekannten Methoden in die optischen Antipoden zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Überführung in diastereomere Salze, z.B. durch Umsetzung eines basischen Endstoffracemats mit einer optisch aktiven Säure, wie Carbonsäure, z.B. Wein-oder Äpfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe und Zwischenprodukte verwendet, welche zu den eingangs als besonders wertvoll geschilderten Verbindungen I führen. Neue Ausgangsstoffe und Zwischenprodukte für die Herstellung der Verbindungen I, ihre Verwendung und ein Verfahren zu ihrer Herstellung bilden ebenfalls einen Gegenstand der Erfindung, wobei die Variablen $R_1$, $R_2$, $R_3$ und $R_4$ die für die Verbindungen I angegebenen Bedeutungen haben.

Die Verbindungen der Formel I und ihre pharmazeutisch verwendbaren Salze können, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Antihypertensiva, verwendet werden.

Die Erfindung betrifft daher gleichfalls pharmazeutische Präparate, die eine Verbindung I in freier Form oder in Form eines pharmazeutisch verwendbaren Salzes als Wirkstoff enthalten, sowie ein Verfahren zu ihrer Herstellung. Bei diesen pharmazeutischen Präparaten handelt es sich um solche zur enteralen, wie oralen, ferner rektalen oder parenteralen Verabreichung an Warmblüter, wobei der pharmakologische Wirkstoff allein oder zusammen mit üblichen pharmazeutischen Hilfsstoffen enthalten ist. Die pharmazeutischen Präparate enthalten z.B. von etwa 0,1 % bis 100 %, vorzugsweise von etwa 1 % bis etwa 60 %, des Wirkstoffs. Pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren, hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragantgummi, Methylcellulose und/oder Polyvinylpyrrolidon, und, wenn erwünscht, Sprengmittel, wie die obengenannten Stärken, ferner Carboxymethylstärke, quervernetztes Polyvinylpyrrolidon, Agar oder Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine sowie weiche, geschlossene Kapseln aus Gelatine und einem Weichmacher, wie Glycerin oder Sorbitol. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole und höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole und Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet, oder

wässrige Injektionssuspensionen, welche viskositätserhöhende Stoffe, z.B. Natriumcarboxymethylcellulose, Sorbit und/oder Dextran, und gegebenenfalls auch Stabilisatoren enthalten.

Die Dosierung des Wirkstoffes kann von verschiedenen Faktoren, wie Applikationsweise, Warmblüter-Spezies, Alter und/oder individuellem Zustand, abhängen. Im Normalfall ist für einen etwa 75 kg schweren Patienten bei oraler Applikation eine ungefähre Tagesdosis von etwa 10 mg bis etwa 250 mg zu veranschlagen.

Die nachfolgenden Beispiele illustrieren die oben beschriebene Erfindung; sie sollen jedoch diese in ihrem Umfang in keiner Weise einschränken. Temperaturen sind in Grad Celsius angegeben. "THF" steht für "Tetrahydrofuran".

Beispiel 1:

6-(n-Butyl)-5-(2′-cyanobiphenyl-4-ylmethyl)-4-hydroxy-2-methyl-pyrimidin (910 mg, 2.54 mmol) und Tributylzinnazid (1.68 g, 5.08 mmol) in o-Xylol (30 ml) werden 24 Stunden unter Rückfluss gerührt. Das Reaktionsgemisch wird im Vakuum eingedampft, der Rückstand mit $CH_2Cl_2/CH_3OH/NH_3$ (5:3:1,50 ml) versetzt und das Gemisch 30 Minuten gerührt. Nach erneutem Eindampfen im Vakuum wird der Rückstand mittels Flash-Chromatographie (Kieselgel 60,40-63 µm, $CH_2Cl_2/CH_3OH/NH_3$ = 160:10:1) aufgetrennt. Man erhält so das 6-(n-Butyl)-4-hydroxy-2-methyl-5-[2′-(1H-tetrazol-5-yl)biphenyl-4-ylmethyl]-pyrimidin als amorphen Festkörper, welcher aus Essigester kristallisiert. Smp.: 236° C (Zersetzung).

Das Ausgangsmaterial kann z. B. wie folgt hergestellt werden:

a) Zu einer Lösung von 3-Oxoheptansäureethylester (9.5 g, 55.1 mmol) in absolutem THF (100 ml) wird bei Raumtemperatur NaH (80 % in Weissoel, 2.4 g, 55.1 mmol) portionsweise zugegeben. Nach beendeter Zugabe wird 30 Minuten gerührt und anschliessend eine Lösung von 4-Brommethyl-2′-cyano-biphenyl (15.0 g, 55.1 mmol) in absolutem THF (150 ml) zugetropft. Das Reaktionsgemisch wird 12 Stunden bei Raumtemperatur gerührt und anschliessend im Vakuum eingedampft. Der Rückstand wird zwischen Essigester und $H_2O$ verteilt und die organische Phase mit $H_2O$ und gesättigter NaCl-Lösung gewaschen, getrocknet ($Na_2SO_4$) und im Vakuum eingedampft. Flash-Chromatographie (Kieselgel 60, 40-63 µm, Hexan/Essigester = 9:1) ergibt den 2-(2′-Cyanobiphenyl-4-ylmethyl)-3-oxo-heptansäureethylester als Oel, welches direkt weiterverarbeitet wird.

b) Zu einer Lösung von Natriummethanolat (3.22 g, 57.8 mmol) in absolutem Methanol (45 ml) wird bei Raumtemperatur Acetamidin-hydrochlorid (3.64 g, 38.5 mmol) zugegeben. Nach 10 Minuten wird eine Lösung von 2-(2′-Cyanobiphenyl-4-ylmethyl)-3-oxo-heptansäureethylester (7.0 g, 19.26 mmol) in absolutem Methanol (30 ml) zugetropft. Die gelbe Suspension wird 24 Stunden bei 50° C gerührt und anschliessend im Vakuum eingedampft. Der Rückstand wird in $H_2O$ suspendiert und die Suspension mit 1N Salzsäure auf pH 5 angesäuert und sodann mit Essigester extrahiert. Die organische Phase wird mit gesättigter NaCl-Lösung gewaschen, getrocknet ($Na_2SO_4$) und eingedampft. Flash-Chromatographie (Kieselgel 60,40-63 µm, $CH_2Cl_2/CH_3OH$ = 98:2) ergibt 6-(n-Butyl)-5-(2′-cyanobiphenyl-4-ylmethyl)-4-hydroxy-2-methyl-pyrimidin, welches aus Diethylether kristalliert und direkt weiterverarbeitet wird.

Beispiel 2:

Ausgehend von 5-(2′-Cyanobiphenyl-4-ylmethyl)-4-hydroxy-2-methyl-6-(n-propyl)-pyrimidin und Tributylzinnazid erhält man in der in Beispiel 1 beschriebenen Weise 4-Hydroxy-2-methyl-6-(n-propyl)-5-[2′-(1H-tetrazol-5-yl)biphenyl-4-ylmethyl]pyrimidin. Weisse Kristalle aus Essigester. Smp.: 206° C (Zersetzung).

Das Ausgangsmaterial kann beispielsweise wie folgt hergestellt werden:

a) Durch Alkylierung von 3-Oxohexansäureethylester mit 4-Brommethyl-2′-cyano-biphenyl in der in Beispiel 1a) beschriebenen Weise erhält man 2-(2′-Cyanobiphenyl-4-ylmethyl)-3-oxo-hexansäureethylester, welcher durch Flash-Chromatographie (Kieselgel 60, 40-63 µm, Hexan/Essigester = 4:1) gereinigt wird, als Oel. Dieses wird direkt weiterverarbeitet.

b) Durch Umsetzung von 2-(2′-Cyanobiphenyl-4-ylmethyl)-3-oxo-hexansäureethylester mit Acetamidin-hydrochlorid und Natriummethanolat in Methanol in der in Beispiel 1b) beschriebenen Weise erhält man 5-(2′-Cyanobiphenyl-4-ylmethyl)-4-hydroxy-2-methyl-6-(n-propyl)-pyrimidin. Dieses wird direkt weiterverarbeitet.

Beispiel 3:

Ausgehend von 6-(n-Butyl)-5-(2′-cyanobiphenyl-4-ylmethyl)-4-hydroxy-2-(n-propyl)-pyrimidin und Tributylzinnazid erhält man in der in Beispiel 1 beschriebenen Weise 6-(n-Butyl)-4-hydroxy-2-(n-propyl)-5-[2′-(1H-tetrazol-5-yl)biphenyl-4-ylmethyl]-pyrimidin. Weisse Kristalle aus Isopropanol/Diethylether. Smp.: 208°C-210°C.

Das Ausgangsmaterial kann beispielsweise wie folgt hergestellt werden.

a) Durch Umsetzung von 2-(2′-Cyanobiphenyl-4-ylmethyl)-3-oxo-heptansäureethylester mit Butyramidin-hydrochlorid und Natriummethanolat in Methanol in der in Beispiel 1b) beschriebenen Weise erhält man 6-(n-Butyl)-5-(2′-cyanobiphenyl-4-ylmethyl)-4-hydroxy-2-(n-propyl)-pyrimidin, welches durch Flash-Chromatographie (Kieselgel 60, 40-63 µm, $CH_2Cl_2/CH_3OH$ = 98:2) gereinigt wird. Dieses wird direkt weiterverarbeitet.

Beispiel 4:

Ausgehend von 5-(2′-Cyanobiphenyl-4-ylmethyl)-2,6-di-(n-butyl)-4-hydroxypyrimidin und Tributylzinnazid erhält man in der in Beispiel 1 beschriebenen Weise 2,6-Di-(n-butyl)-4-hydroxy-5-[2′-(1H-tetrazol-5-yl)-biphenyl-4- ylmethyl]-pyrimidin. Weisse Kristalle aus Essigester. Smp.: 212-213°C.

Das Ausgangsmaterial kann beispielsweise wie folgt hergestellt werden.

a) Durch Umsetzung von 2-(2′-Cyanobiphenyl-4-ylmethyl)-3-oxo-heptansäureethylester mit Valeramidin-hydrochlorid und Natriummethanolat in Methanol in der in Beispiel 1b) beschriebenen Weise erhält man 5-(2′-Cyanobiphenyl-4-ylmethyl)-2,6-di-(n-butyl)-4-hydroxy-pyrimidin, welches durch Flash-Chromatographie (Kieselgel 60,40-63 µm, $CH_2Cl_2/CH_3OH$ = 98:2) gereinigt wird. Dieses wird direkt weiterverarbeitet.

Beispiel 5:

Ausgehend von 6-(n-Butyl)-4-chlor-5-(2′-cyanobiphenyl-4-ylmethyl)-2-methyl-pyrimidin und Tributylzinnazid erhält man in der in Beispiel 1 beschriebenen Weise 6-(n-Butyl)-4-chlor-2-methyl-5-[2′-( 1H-tetrazol-5-yl)biphenyl-4-ylmethyl]-pyrimidln als amorphen Festkörper. Smp.: 120°C-122°C.

Das Ausgangsmaterial kann beispielsweise wie folgt hergestellt werden.

a) Zu 11 ml $POCl_3$ wird bei 0°C unter Rühren zunächst N,N-Dimethylanilin (1.67 ml, 13.15 mmol) und anschliessend 6-(n-Butyl)-4-hydroxy-2-methyl-5-(2′-cyanobiphenyl-4-ylmethyl)-pyrimidin (4.7 g, 13.15 mmol) zudosiert. Das Reaktionsgemisch wird langsam aufgeheizt und 1,5 Stunden unter Rückfluss gerührt. Das Reaktionsgemisch wird sodann auf Wasser mit einer Temperatur von 20-25°C gegossen. Man lässt 30 Minuten stehen und extrahiert dann mit Diethylether. Die organische Phase wird getrocknet ($Na_2SO_4$) und eingedampft und der Rückstand mittels Flash-Chromatographie (Kieselgel 60,40-63 µm, $CH_2Cl_2$) gereinigt. Man erhält so 6-(n-Butyl)-4-chlor-5-(2′-cyanobiphenyl-4-ylmethyl)-2-methyl-pyrimidin, welches direkt weiterverarbeitet wird.

Beispiel 6:

Ausgehend von 6-(n-Butyl)-5-(2′-cyanobiphenyl-4-ylmethyl)-4-methoxy-2-methyl-pyrimidin und Tributylzinnazid erhält man in der in Beispiel 1 beschriebenen Weise 6-(n-Butyl)-4-methoxy-2-methyl-5-[2′-( 1H-tetrazol-5-yl)biphenyl-4-ylmethyl]-pyrimidin. Weisse Kristalle aus Essigester/Diethylether. Smp.: 113-115°C.

Das Ausgangsmaterial kann beispielsweise wie folgt hergestellt werden.

a) Zu einer Lösung von Natriummethanolat (355 mg, 6.38 mmol) in absolutem Methanol (5 ml) wird 6-(n-Butyl)-4-chlor-5-(2′-cyanobiphenyl-4-ylmethyl)-2-methyl-pyrimidin (1.0 g, 2.66 mmol) in absolutem Methanol (10 ml) zugetropft. Das Reaktionsgemisch wird 24 Stunden bei Raumtemperatur gerührt und anschliessend im Vakuum eingedampft. Der Rückstand wird zwischen Essigester und $H_2O$ verteilt und die organische Phase mit $H_2O$ und gesättigter NaCl-Lösung gewaschen, getrocknet ($Na_2SO_4$) und im Vakuum eingedampft. Das Rohprodukt wird mittels Flash-Chromatographie (Kieselgel 60, 40-63 µm, $CH_2Cl_2/CH_3OH$ = 98:2) gereinigt. Man erhält so 6-(n-Butyl)-5-(2′-cyanobiphenyl-4-ylmethyl)-4-methoxy-2-methyl-pyrimidin welches direkt weiterverarbeitet wird.

Beispiel 7:

Ausgehend von 6-(n-Butyl)-5-(2′-cyanobiphenyl-4-ylmethyl)-4-(2-methoxyethoxy)-2-methyl-pyrimidin und Tributylzinnazid erhält man in der in Beispiel 1 beschriebenen Weise 6-(n-Butyl)-4-(2-methoxyethoxy)-2-methyl--5-[2′-(1H-tetrazol-5-yl)-biphenyl-4-ylmethyl]-pyrimidin. Weisse Kristalle aus Essigester/Hexan. Smp.: 133°C-135°C.

Das Ausgangsmaterial kann beispielsweise wie folgt hergestellt werden:

a) Natrium (122 mg, 5.32 mmol) wird in 10 ml 2-Methoxyethanol bei Raumtemperatur gelöst. Anschliessend wird eine Lösung von 6-(n-Butyl)-4-chlor-5-(2′-cyanobiphenyl-4-ylmethyl)-2-methyl-pyrimidin (1.0 g, 2.66 mmol) in 2-Methoxyethanol (5 ml) zugetropft. Das Reaktionsgemisch wird 24 Stunden bei Raumtemperatur gerührt und anschliessend im Vakuum eingedampft. Der Rückstand wird zwischen Essigester und $H_2O$ verteilt und die organische Phase mit $H_2O$ und gesättigter NaCl-Lösung gewaschen, getrocknet ($Na_2SO_4$) und im Vakuum eingedampft. Das Rohprodukt wird mittels Flash-Chromatographie (Kieselgel 60,40-63 µm, $CH_2Cl_2/CH_3OH$ = 98:2) gereinigt. Man erhält so 6-(n-Butyl)-5-(2′-cyanobiphenyl-4-ylmethyl)-4(2-methoxyethoxy)-2-methyl-pyrimidin, welches direkt weiterverarbeitet wird.

Beispiel 8:

Ausgehend von 6-(n-Butyl)-5-(2′-cyanobiphenyl-4-ylmethyl)-4-dimethylamino-2-methyl-pyrimidin und Tributylzinnazid erhält man in der in Beispiel 1 beschriebenen Weise 6-(n-Butyl)-4-dimethylamino-2-methyl--5[2′-(1H-tetrazol-5-yl)biphenyl-4-ylmethyl]-pyrimidin. Weisse Kristalle aus Aceton. Smp.: 195°C-197°C.

Das Ausgangsmaterial kann beispielsweise wie folgt hergestellt werden:

a) Zu einer Lösung von 6-(n-Butyl)-4-chlor-5-(2′-cyanobiphenyl-4-ylmethyl)-2-methyl-pyrimidin (1.0 g, 2.66 mmol) in Ethanol (10 ml) wird Dimethylamin (33 % in Ethanol, 1.9 ml) zugegeben. Das Reaktionsgemisch wird 24 Stunden unter Rückfluss gerührt und anschliessend im Vakuum eingedampft. Der Rückstand wird zwischen Essigester und $H_2O$ verteilt und die organische Phase mit $H_2O$ und gesättigter NaCl-Lösung gewaschen, getrocknet ($Na_2SO_4$) und im Vakuum eingedampft. Das Rohprodukt wird mittels Flash-Chromatographie (Kieselgel 60,40-63 µm, $CH_2Cl_2/CH_3OH$ = 98:2) gereinigt. Man erhält so 6-(n-Butyl)-5-(2′-cyanobiphenyl-4-ylmethyl)-4-dimethylamino-2-methyl-pyrimidin, welches direkt weiterverarbeitet wird.

Beispiel 9:

Ausgehend von 6-(n-Butyl)-5-(2′-cyanobiphenyl-4-ylmethyl)-4-hydroxy-2-isopropyl-pyrimidin und Tributylzinnazid erhält man in der in Beispiel 1 beschriebenen Weise 6-(n-Butyl)-4-hydroxy-2-isopropyl-5-[2′-(1H-tetrazol-5-yl)biphenyl-4-ylmethyl]-pyrimidin. Smp.: 234-236°C (aus Isopropanol/Diethylether; Zersetzung).

Das Ausgangsmaterial kann beispielsweise wie folgt hergestellt werden:

a) Durch Umsetzung von 2-(2′-cyanobiphenyl-4-ylmethyl)-3-oxo-heptansäureethylester mit Isobutyramidin-hydrochlorid und Natriummethanolat in Methanol in der in Beispiel 1b) beschriebenen Weise erhält man 6-(n-Butyl)-5-(2′-cyanobiphenyl-4-ylmethyl)-4-hydroxy-2-isopropyl-pyrimidin, welches durch Flash-Chromatographie (Kieselgel 60,40-63 µm, $CH_2Cl_2/CH_3OH$ = 98:2) gereinigt und direkt weiterverarbeitet wird.

Beispiel 10:

Zu einer Suspension von 2,6-Di-(n-butyl)-4-hydroxy-5-[2′-(1H-tetrazol-5-yl)-biphenyl-4-ylmethyl]-pyrimidin (1,2 g, 2,7 mmol) in 60 ml Ethanol werden 2,7 ml einer 1N Lösung von KOH in Ethanol zugegeben. Die entstandene Lösung wird im Vakuum eingedampft. Nach Zugabe von Isopropanol tritt Kristallisation ein. Die Kristalle werden abfiltriert und bei 100°C im Vakuum getrocknet. Man erhält so das 2,6-Di-(n-butyl)-4-hydroxy-5-[2′-(1H-tetrazol-5-yl)biphenyl-4-ylmethyl]-pyrimidin-Kaliumsalz. Smp. 276-278°C (Zersetzung).

Beispiel 11:

Ausgehend von 2,6-Di-(n-butyl)-4-hydroxy-5-[2′-(1H-tetrazol-5-yl)biphenyl-4-ylmethyl]-pyrimidin und 1N NaOH in Ethanol erhält man in der in Beispiel 10 beschriebenen Weise das 2,6-Di-(n-butyl)-4-hydroxy-5-[2′-(1H-tetrazol-5-yl)biphenyl-4-ylmethyl]-pyrimidin-Natriumsalz, welches aus Acetonitril kristallisiert wird. Smp. 333-335°C (Zersetzung).

Beispiel 12:

Ausgehend von 6-(But-1-en-4-yl)-2-(n-butyl)-5-(2′-cyanobiphenyl-4-ylmethyl)-4-hydroxy-pyrimidin und Tributylzinnazid erhält man in der in Beispiel 1 beschriebenen Weise 6-(But-1-en-4-yl)-2-(n-butyl)-4-hydroxy-5-[2′-(1H-tetrazol-5-yl)biphenyl-4-ylmethyl]-pyrimidin. Smp.: 203-205° C (aus Essigsäure/$H_2O$; Zersetzung).

Das Ausgangsmaterial kann beispielsweise wie folgt hergestellt werden:

a) Durch Alkylierung von 3-Oxohept-6-ensäureethylester mit 4-Brommethyl-2′-cyanobiphenyl in der in Beispiel 1a) beschriebenen Weise erhält man den 2-(2′-Cyanobiphenyl-4-ylmethyl)-3-oxohept-6-ensäureethylester, welcher durch Flash-Chromatographie (Kieselgel 60,40-63 µm, Hexan/Essigester = 9:1) gereinigt und direkt weiterverarbeitet wird.

b) Durch Umsetzung von 2-(2′-Cyanobiphenyl-4-ylmethyl)-3-oxohept-6-ensäureethylester mit Valeramidin-hydrochlorid und Natriummethanolat in Methanol in der in Beispiel 1b) beschriebenen Weise erhält man 6-(But-1-en-4-yl)-2-(n-butyl)-5-(2′-cyanobiphenyl-4-ylmethyl)-4-hydroxy-pyrimidin, welches durch Flash-Chromatographie (Kieselgel 60,40-63 µm, Essigester/Hexan = 1:1) gereinigt und direkt weiterverarbeitet wird.

Beispiel 13:

Ausgehend von 4-Amino-6-(n-butyl)-5-(2′-cyanobiphenyl-4-ylmethyl)-2-methyl-pyrimidin und Tributylzinnazid erhält man in der in Beispiel 1 beschriebenen Weise 4-Amino-6-(n-butyl)-2-methyl-5-[2′-(1H-tetrazol-5-yl)biphenyl-4-ylmethyl]-pyrimidin. Smp.: 177-179° C (aus Methanol; Zersetzung).

Das Ausgangsmaterial kann beispielsweise wie folgt hergestellt werden:

a) 6-(n-Butyl)-4-chlor-5-(2′-cyanobiphenyl-4-ylmethyl)-2-methyl-pyrimidin (0,2 g, 0,53 mmol) und 2 ml $NH_3$ in 10 ml Ethanol werden 4 Tage im Bombenrohr bei 120° C gehalten. Nach dem Eindampfen im Vakuum wird der Rückstand mittels Flash-Chromatographie (Kieselgel 60,40-63 µm, $CH_2Cl_2/CH_3OH$ /= 95:5) gereinigt. Man erhält so 4-Amino-6(n-butyl)-5-(2′-cyanobiphenyl-4-ylmethyl)-2-methyl-pyrimidin, welches direkt weiterverarbeitet wird.

Beispiel 14:

Ausgehend von 6-(n-Butyl)-5-(2′-cyanobiphenyl-4-ylmethyl)-2-methyl-4-methylthio-pyrimidin und Tributylzinnazid erhält man in der in Beispiel 1 beschriebenen Weise 6-(n-Butyl)-2-methyl-4-methylthio--5-[2′-(1H-tetrazol-5-yl)biphenyl-4-ylmethyl]-pyrimidin. Smp.: 176-178° C (aus Essigester; Zersetzung).

Das Ausgangsmaterial kann beispielsweise wie folgt hergestellt werden:

a) 6-(n-Butyl)-4-chlor-5-(2′-cyanobiphenyl-4-ylmethyl)-2-methyl-pyrimidin (1,0 g, 2,66 mmol) und Natriummethanthiolat (0,21 g, 2,92 mmol) werden in 10 ml 1,3-Dimethyl-3,4,5,6-tetrahydro-2(1H)-pyrimidinon 6 Stunden bei Raumtemperatur gerührt. Das Reaktionsgemisch wird auf $H_2O$ gegossen und mit Essigester extrahiert. Die organische Phase wird mit $H_2O$ gründlich gewaschen, getrocknet ($Na_2SO_4$) und im Vakuum eingedampft. Flash-Chromatographie (Kieselgel 60,40-63 µm, Hexan/Essigester = 4:1) ergibt 6-(n-Butyl)-5-(2′-cyanobiphenyl-4-ylmethyl)-2-methyl-4-methylthio-pyrimidin, welches direkt weiterverarbeitet wird.

Beispiel 15:

Ausgehend von 6-(n-Butyl)-5-(2′-cyanobiphenyl-4-ylmethyl)-2-ethyl-4-hydroxy-pyrimidin und Tributylzinnazid erhält man in der in Beispiel 1 beschriebenen Weise 6-(n-Butyl)-2-ethyl-4-hydroxy-5-[2′-(1H-tetrazol-5-yl)biphenyl-4-ylmethyl]-pyrimidin. Smp.: 235-237° C (aus Isopropanol; Zersetzung).

Das Ausgangsmaterial kann beispielsweise wie folgt hergestellt werden:

a) Durch Umsetzung von 2-(2′-Cyanobiphenyl-4-ylmethyl)-3-oxo-heptansäureethylester mit Propionamidin-hydrochlorid und Natriummethanolat in Methanol in der in Beispiel 1b) beschriebenen Weise erhält man 6-(n-Butyl)-5-(2′-cyanobiphenyl-4-ylmethyl)-2-ethyl-4-hydroxy-pyrimidin, welches durch Flash-Chromatographie (Kieselgel 60,40-63 µm, $CH_2Cl_2/CH_3OH$ = 98:2) gereinigt und direkt weiterverarbeitet wird.

Beispiel 16:

Ausgehend von 6-(n-Butyl)5-(2′-cyanobiphenyl-4-ylmethyl)-4-(2-hydroxyethoxy)-2-methyl-pyrimidin und Tributylzinnazid erhält man in der in Beispiel 1 beschriebenen Weise 6-(n-Butyl)-4-(2-hydroxyethoxy)-2-methyl-5-[2′-(1H-tetrazol-5-yl)-biphenyl-4-ylmethyl]-pyrimidin. Smp.: 168-170 °C (aus Essigester; Zersetzung).

Das Ausgangsmaterial kann beispielsweise wie folgt hergestellt werden:

a) Zu einer Lösung von Natrium (0,12 g, 5,3 mmol) in 20 ml Ethylenglykol wird 6-(n-Butyl)-4-chlor-5-(2′-cyanobiphenyl-4-ylmethyl)-2-methyl-pyrimidin (1,0 g, 2,6 mmol) zugegeben. Das Reaktionsgemisch wird 3 Stunden bei 100 °C gerührt. Nach dem Eindampfen im Vakuum wird der Rückstand zwischen Essigester und $H_2O$ verteilt und die organische Phase über $Na_2SO_4$ getrocknet und im Vakuum eingedampft. Flash-Chromatographie (Kieselgel 60,40-63 µm, $CH_2Cl_2/CH_3OH$ = 95:5) ergibt 6-(n-Butyl)-5-(2′-cyanobiphenyl-4-ylmethyl)-4-(2-hydroxyethoxy)-2-methyl-pyrimidin, welches direkt weiterverarbeitet wird.

Beispiel 17:

Ausgehend von 6-(n-Butyl)-5-(2′-cyanobiphenyl-4-ylmethyl)-4-ethoxycarbonylmethoxy-2-methyl-pyrimidin und Tributylzinnazid erhält man in der in Beispiel 1 beschriebenen Weise 6-(n-Butyl)-4-ethoxycarbonylmethoxy-2-methyl-5-[2′-(1H-tetrazol-5-yl)biphenyl-4-ylmethyl]-pyrimidin als amorphen Festkörper.

Das Ausgangsmaterial kann beispielsweise wie folgt hergestellt werden:

a) Zu einer Lösung von 6-(n-Butyl)-5-(2′-cyanobiphenyl-4-ylmethyl)-4-hydroxy-2-methyl-pyrimidin (2,0 g, 5,6 mmol) in 50 ml N,N-Dimethylformamid wird NaH (0,17 g, 5,6 mmol, 80 % in Weissöl) zugegeben. Das Reaktionsgemisch wird 30 Minuten bei Raumtemperatur gerührt. Anschliessend wird Bromessigsäureethylester (0,81 ml, 7,3 mmol) zugegeben. Nach 3 Stunden wird im Vakuum eingedampft und der Rückstand zwischen Essigester und $H_2O$ verteilt. Die organische Phase wird mit $Na_2SO_4$ getrocknet und im Vakuum eingedampft. Flash-Chromatographie (Kieselgel 60,40-63 µm, Hexan/Essigester = 4:1) ergibt 6-(n-Butyl)5-(2′-cyanobiphenyl-4-ylmethyl)-4-ethoxycarbonylmethoxy-2-methyl-pyrimidin, welches direkt weiterverarbeitet wird.

Beispiel 18:

Ausgehend von 5-[3-Brom-2′-cyano-biphenyl-4-ylmethyl]-2,6-di-(n-butyl)-4-hydroxy-pyrimidin und Tributylzinnazid erhält man in der in Beispiel 1 beschriebenen Weise 5-[3-Brom-2′-(1H-tetrazol-5-yl)-biphenyl-4-ylmethyl]-2,6-di-(n-butyl)-4-hydroxy-pyrimidin. Smp.: 222-224 °C (aus Isopropanol/Diethylether, Zersetzung).

Das Ausgangsmaterial kann beispielsweise wie folgt hergestellt werden:

a) Zu einer Suspension von $AlCl_3$ (21,0 g, 0,157 mol) in Tetrachlorethan wird 4-Methyl-2′-cyano-biphenyl zugegeben. Unter Rühren wird auf 60 °C erwärmt. Zur entstandenen Lösung wird eine Lösung von Brom (20,7 g, 0,13 mol) in 100 ml Tetrachlorethan bei 60 °C zugetropft und das Reaktionsgemisch 24 Stunden gerührt. Nach Zusatz von weiteren 6,2 g $AlCl_3$ und Erwärmen auf 70 °C lässt sich dünnschichtchromatographisch (Toluol) kein Edukt mehr feststellen. Das Reaktionsgemisch wird sodann unter Eiskühlung mit 200 ml konzentrierter Salzsäure zersetzt und die organische Phase abgetrennt und im Vakuum eingedampft. Der Rückstand wird in Essigester gelöst und die Lösung mit $H_2O$ und Natriumcarbonat-Lösung gewaschen, getrocknet ($MgSO_4$) und im Vakuum eingedampft. Flash-Chromatographie (Kieselgel 60,40-63 µm, Hexan/Essigester = 4:1) ergibt 3-Brom-2′-cyano-4-methyl-biphenyl, welches aus Cyclohexan umkristallisiert wird. Smp.: 104-106 °C.

b) Zu einer Lösung von 3-Brom-2′-cyano-4-methyl-biphenyl (8,9 g, 0,033 mol) in 900 ml Tetrachlorethan wird nach Zugabe von Benzoylperoxid (0,1 g) unter UV-Bestrahlung bei 100-110 °C eine Lösung von Brom (5,6 g, 0,035 mol) in 20 ml Tetrachlorethan zugetropft. Nach 30 Minuten wird das Reaktionsgemisch abgekühlt und im Vakuum eingedampft. Umkristallisation des Rückstands aus Essigester ergibt 3-Brom-4-brommethyl-2′-cyano-biphenyl. Smp.: 152-153 °C.

c) Durch Alkylierung von 3-Oxoheptansäureethylester mit 3-Brom-4-brommethyl-2′-cyano-biphenyl in der in Beispiel 1a) beschriebenen Weise erhält man 2-(3-Brom-2′-cyano-biphenyl-4-ylmethyl)-3-oxo-heptansäureethylester, welcher mittels Flash-Chromatographie (Kieselgel 60,40-63 µm, Hexan/Essigester = 9:1) gereinigt und direkt weiterverarbeitet wird.

d) Durch Umsetzung von 2-(3-Brom-2′-cyano-biphenyl-4-ylmethyl)-3-oxo-heptansäureethylester mit Valeramidin-hydrochlorid und Natriummethanolat in Methanol in der in Beispiel 1b) beschriebenen Weise erhält man 5-[3-Brom-2′-cyano-biphenyl-4-ylmethyl]-2,6-di-(n-butyl)-4-hydroxy-pyrimidin, welches mittels

Flash-Chromatographie (Kieselgel 60,40-63 µm, $CH_2Cl_2/CH_3OH$ = 98:2) gereinigt und direkt weiterverarbeitet wird.

Beispiel 19:

Ausgehend von 5-(3′-Cyanobiphenyl-4-ylmethyl)-2,6-di-(n-butyl)-4-hydroxy-pyrimidin und Tributylzinnazid erhält man in der in Beispiel 1 beschriebenen Weise 2,6-Di-(n-butyl)-4-hydroxy-5-[3′-(1H-tetrazol-5-yl)-biphenyl-4-ylmethyl]-pyrimidin als amorphen Festkörper.

Das Ausgangsmaterial kann beispielsweise wie folgt hergestellt werden:

a) 4-Methylphenyl-trimethyl-zinn (5,9 g, 20 mmol), 3-Iodbenzonitril (5,0 g, 22 mmol) und Tetrakis-(triphenylphosphan)palladium (1,16 g, 1 mmol) in 60 ml o-Xylol werden 12 Stunden unter Rückfluss gerührt. Nach dem Abkühlen auf Raumtemperatur wird die schwarze Suspension filtriert und das Filtrat im Vakuum eingedampft. Der Rückstand wird in Essigester gelöst und die Lösung mit einer Lösung von Kaliumfluorid in $H_2O$ (10 %) gewaschen, mit $Na_2SO_4$ getrocknet und im Vakuum eingedampft. Flash-Chromatographie (Kieselgel 60,40-63 µm, Hexan/Essigester = 9:1) ergibt 3′-Cyano-4-methyl-biphenyl, welches direkt weiterverarbeitet wird.

b) 3′-Cyano-4-methyl-biphenyl (1,8 g, 9,3 mmol), N-Bromsuccinimid (1,66 g, 9,3 mmol) und α,α′-Azoisobutyronitril (20 mg) in 30 ml Tetrachlormethan werden 6 Stunden unter Rückfluss gerührt. Nach dem Eindampfen des Reaktionsgemisches im Vakuum wird der Rückstand in Essigester gelöst und die Lösung mit $H_2O$ gewaschen, getrocknet ($Na_2SO_4$) und im Vakuum eingedampft. Kristallisation aus Essigester/Hexan ergibt 4-Brommethyl-3′-cyano-biphenyl. Smp.: 106-108° C.

c) Durch Alkylierung von 3-Oxoheptansäureethylester mit 4-Brommethyl-3′-cyano-biphenyl in der in Beispiel 1a) beschriebenen Weise erhält man 2-(3′-Cyanobiphenyl-4-ylmethyl)-3-oxo-heptansäureethylester, welcher mittels Flash-Chromatographie (Kieselgel 60,40-63 µm, Hexan/Essigester = 9:1) gereinigt und direkt weiterverarbeitet wird.

d) Durch Umsetzung von 2-(3′-Cyanobiphenyl-4-ylmethyl)-3-oxo-heptansäureethylester mit Valeramidin-hydrochlorid und Natriummethanolat in Methanol in der in Beispiel 1b) beschriebenen Weise erhält man 5-(3′-Cyanobiphenyl-4-ylmethyl)-2,6-di-(n-butyl)-4-hydroxy-pyrimidin, welches mittels Flash-Chromatographie (Kieselgel 60,40-63 µm, $CH_2Cl_2/CH_3OH$ = 98:2) gereinigt und direkt weiterverarbeitet wird.

Beispiel 20:

Ausgehend von 5-(2′-Cyanobiphenyl-3-ylmethyl)-2,6-di-(n-butyl)-4-hydroxy-pyrimidin und Tributylzinnazid erhält man in der in Beispiel 1 beschriebenen Weise 2,6-Di-(n-butyl)-4-hydroxy-5-[2′-(1H-tetrazol-5-yl)-biphenyl-3-ylmethyl]-pyrimidin. Smp.: 185-187° C (aus Isopropanol/Diethylether, Zersetzung).

Das Ausgangsmaterial kann beispielsweise wie folgt hergestellt werden:

a) Durch Umsetzung von 3-Methylphenyl-trimethyl-zinn mit 2-Iodbenzonitril in der in Beispiel 19a) beschriebenen Weise erhält man 2′-Cyano-3-methyl-biphenyl, welches mittels Flash-Chromatographie (Kieselgel 60,40-63 µm, Hexan/Essigester = 9:1) gereinigt und direkt weiterverarbeitet wird.

b) Durch Umsetzung von 2′-Cyano-3-methyl-biphenyl mit N-Bromsuccinimid in der in Beispiel 19b) beschriebenen Weise erhält man 3-Brommethyl-2′-cyano-biphenyl, welches mittels Säulenchromatographie (Kieselgel 60,40-63 µm, Hexan/Essigester = 8:1) gereinigt und direkt weiterverarbeitet wird.

c) Durch Alkylierung von 3-Oxoheptansäureethylester mit 3-Brommethyl-2′-cyano-biphenyl in der in Beispiel 1a) beschriebenen Weise erhält man 2-(2′-Cyanobiphenyl-3-ylmethyl)-3-oxo-heptansäureethylester, welcher mittels Flash-Chromatographie (Kieselgel 60,40-63 µm, Hexan/Essigester = 9:1) gereinigt und direkt weiterverarbeitet wird.

d) Durch Umsetzung von 2-(2′-Cyanobiphenyl-3-ylmethyl)-3-oxo-heptansäureethylester mit Valeramidin-hydrochlorid und Natriummethanolat in Methanol in der in Beispiel 1b) beschriebenen Weise erhält man 5-(2′-Cyanobiphenyl-3-ylmethyl)-2,6-di-(n-butyl)-4-hydroxy-pyrimidin, welches mittels Säulenchromatographie (Kieselgel 60,40-63 µm, $CH_2Cl_2/CH_3OH$ = 98:2) gereinigt und direkt weiterverarbeitet wird.

Beispiel 21:

Ausgehend von 5-[2-(2′-Cyanobiphenyl-4-yl)ethyl]-2,6-di-(n-butyl)-4-hydroxy-pyrimidin und Tributylzinnazid erhält man in der in Beispiel 1 beschriebenen Weise 2,6-Di-(n-butyl)-4-hydroxy-5-[2-(2′-(1H-tetrazol-5-yl)biphenyl-4-yl)ethyl]-pyrimidin. Smp.: 217-218° C (aus Isopropanol/Essigester, Zersetzung).

Das Ausgangsmaterial kann beispielsweise wie folgt hergestellt werden:

a) Zu einer Lösung von Methoxymethyl-triphenyl-phosphoniumchlorid (2,57 g, 7,5 mmol) in 25 ml wasserfreiem Tetrahydrofuran wird bei Raumtemperatur eine Lösung von Lithium-bis(trimethylsilyl)-amid in Tetrahydrofuran (1 M, 7,5 ml, 7,5 mmol) zugetropft. Nach 30 Minuten wird unter Eiskühlung eine Lösung von 2′-Cyano-4-formyl-biphenyl (1,04 g, 5 mmol) in 30 ml Tetrahydrofuran zugetropft und das Reaktionsgemisch anschliessend 30 Minuten bei Raumtemperatur gerührt. Das Reaktionsgemisch wird in 150 ml eiskalte gesättigte Ammoniumchlorid-Lösung gegossen und mit Diethylether extrahiert. Die organische Phase wird mit $H_2O$ gewaschen, getrocknet ($Na_2SO_4$) und im Vakuum eingedampft. Der Rückstand wird mittels Flash-Chromatographie (Kieselgel 60, 40-63 µm, Hexan/Essigester = 4:1) gereinigt. Man erhält so 2′-Cyano-4-(2-methoxyethenyl)-biphenyl, welches direkt weiterverarbeitet wird.

b) 2′-Cyano-4-(2-methoxyethenyl)-biphenyl (8,2 g, 34,9 mmol) wird in 120 ml Ameisensäure (95 %) suspendiert und die Suspension 30 Minuten bei Raumtemperatur gerührt. Die entstandene Lösung wird mit 1,5l $H_2O$ verdünnt und das Gemisch mit Diethylether extrahiert. Die organische Phase wird mit gesättigter $NaHCO_3$-Lösung gewaschen, getrocknet ($MgSO_4$) und im Vakuum eingedampft Der Rückstand wird mittels Flash-Chromatographie (Kieselgel 60,40-63 µm, Hexan/Essigester = 2:1) gereinigt. Man erhält so 2′-Cyano-4-(2-oxoethyl)-biphenyl, welches direkt weiterverarbeitet wird.

c) Zu einer Lösung von 2′-Cyano-4-(2-oxoethyl)-biphenyl (2,5 g, 11,3 mmol) in 50 ml Methanol wird portionsweise unter Rühren $NaBH_4$ (0,86 g, 22,62 mmol) unter Eiskühlung zugegeben. Nach 30 Minuten Rühren bei 0° C wird das Reaktionsgemisch im Vakuum eingedampft und der Rückstand zwischen Diethylether und $H_2O$ verteilt. Die organische Phase wird mit $H_2O$ gewaschen, getrocknet ($Na_2SO_4$) und im Vakuum eingedampft. Nach Flash-Chromatographie (Kieselgel 60,40-63 µm, Hexan/Essigester = 1:1) erhält man 2′-Cyano-4-(2-hydroxyethyl)-biphenyl, welches direkt weiterverarbeitet wird.

d) Zu einer Lösung von 2′-Cyano-4-(2-hydroxyethyl)-biphenyl (2,23 g, 10 mmol) und Triphenylphosphan (2,9 g, 11 mmol) in 50 ml $CH_2Cl_2$ wird unter Rühren bei 0° C N-Bromsuccinimid (1,96 g, 11 mmol) zugegeben. Das Reaktionsgemisch wird 12 Stunden bei Raumtemperatur gerührt und anschliessend im Vakuum eingedampft. Nach Flash-Chromatographie (Kieselgel 60,40-63 µm, Hexan/Essigester = 4:1) erhält man 4-(2-Bromethyl)-2′-cyano-biphenyl, welches direkt weiterverarbeitet wird.

e) Durch Alkylierung von 3-Oxoheptansäureethylester mit 4-(2-Bromethyl)-2′-cyano-biphenyl in der in Beispiel 1a) beschriebenen Weise erhält man 2-[2-(2′-Cyanobiphenyl-4-yl)ethyl]-3-oxo-heptansäureethylester, welcher mittels Flash-Chromatographie (Kieselgel 60,40-63 µm, Hexan/Essigester = 6:1) gereinigt und direkt weiterverarbeitet wird.

f) Durch Umsetzung von 2-[2-(2′-Cyanobiphenyl-4-yl)ethyl]-3-oxo-heptansäureethylester mit Valeramidin-hydrochlorid und Natriummethanolat in Methanol in der in Beispiel 1b) beschriebenen Weise erhält man 5-[2-(2′-Cyanobiphenyl-4-yl)ethyl]-2,6-di-(n-butyl)-4-hydroxy-pyrimidin, welches mittels Flash-Chromatographie (Kieselgel 60,40-63 µm, $CH_2Cl_2/CH_3OH$ = 95:5) gereinigt wird. Smp.: 166-167° C.

Beispiel 22:

Ausgehend von 6-(n-Butyl)-5-(2′-cyanobiphenyl-4-ylmethyl)-4-hydroxy-pyrimidin und Tributylzinnazid erhält man in der in Beispiel 1 beschriebenen Weise 6-(n-Butyl)-4-hydroxy-5-[2′-(1H-tetrazol-5-yl)biphenyl-4-ylmethyl]-pyrimidin. Smp.: 215-219° C (aus Ethanol, Zersetzung).

Das Ausgangsmaterial kann beispielsweise wie folgt hergestellt werden:

Durch Umsetzung von 2-(2′-Cyanobiphenyl-4-ylmethyl)-3-oxo-heptansäureethylester mit Formamidin-hydrochlorid und Natriummethanolat in Methanol in der in Beispiel 1b) beschriebenen Weise erhält man 6-(n-Butyl)-5-(2′-cyanobiphenyl-4-ylmethyl)-4-hydroxy-pyrimidin. Smp.: 169-171° C (aus Essigester).

Beispiel 23:

6-(n-Butyl)-5-(2′-cyanobiphenyl-4-ylmethyl)-4-methoxycarbonyl-2-methyl-pyrimidin (3,0 g, 7,5 mmol) und Tributylzinnazid (12,46 g, 37,5 mmol) in o-Xylol (75 ml) werden 48 Stunden bei 120° C gerührt. Nach dem Eindampfen des Reaktionsgemisches im Vakuum wird der Rückstand in Diethylether gelöst und in die Lösung Chlorwasserstoff eingeleitet. Der entstandene Niederschlag wird abfiltriert und sodann in 1N Kalilauge gelöst. Die Lösung wird mit Diethylether extrahiert, mit 2N Salzsäure angesäuert und anschliessend mit Essigester extrahiert. Die organische Phase wird mit gesättigter NaCl-Lösung gewaschen,

getrocknet ($Na_2SO_4$) und im Vakuum eingedampft. Flash-Chromatographie (Kieselgel 60,40-63 µm, $CH_2Cl_2/CH_3OH/NH_3$ = 40:10:1) ergibt 6-(n-Butyl)-4-carboxy-2-methyl-5-[2′-(1H-tetrazol-5-yl)biphenyl-4-ylmethyl]-pyrimidin [Smp.: 120-122° (aus Isopropanol/Wasser)].

Das Ausgangsmaterial kann beispielsweise wie folgt hergestellt werden:

a) 6-(n-Butyl)-4-chlor-5-(2′-cyanobiphenyl-4-ylmethyl)-2-methyl-pyrimidin (16,0 g, 42,6 mmol), Tributyl-vinyl-zinn (14,9 ml, 51,1 mmol) und Bis(triphenylphosphan)palladium(II)-dichlorid (0.57 g, 0,82 mmol) in Dimethylformamid (160 ml) werden 2 Stunden bei 90° C gerührt. Nach dem Abkühlen auf Raumtemperatur wird das Reaktionsgemisch mit $H_2O$ verdünnt und mit Diethylether extrahiert. Die organische Phase wird dreimal mit Kaliumfluorid-Lösung (10 %) gewaschen, getrocknet ($Na_2SO_4$) und im Vakuum eingedampft. Der Rückstand wird mittels Flash-Chromatographie (Kieselgel 60,40-63 µm, Hexan/Essigester = 3:1) gereinigt und das Produkt anschliessend aus Diisopropylether/Hexan umkristallisiert. Man erhält so 6-(n-Butyl)-5-(2′-cyanobiphenyl-4-ylmethyl)-2-methyl-4-vinyl-pyrimidin (Smp.: 115-117°).

b) In eine Lösung von 6-(n-Butyl)-5-(2′-cyanobiphenyl-4-ylmethyl)-2-methyl-4-vinyl-pyrimidin (14,6 g, 39,7 mmol) in $CH_2Cl_2$ (275 ml) und Methanol (275 ml) wird bei -70° C Ozon eingeleitet, bis eine Blaufärbung bestehen bleibt. Man rührt das Reaktionsgemisch noch 30 Minuten bei -70° C und leitet anschliessend Argon hindurch, bis das Gemisch farblos wird. Anschliessend werden Dimethylsulfid (29 ml, 0,4 mol) und $NaHCO_3$ (0,5 g) zugegeben. Das Kältebad wird entfernt und das Reaktionsgemisch 12 Stunden bei Raumtemperatur gerührt. Nach dem Eindampfen des Reaktionsgemisches im Vakuum und Flash-Chromatographie des Rückstandes (Kieselgel 60,40-63 µm, Hexan/Essigester = 4:1) erhält man 6-(n-Butyl)-5-(2′-cyanobiphenyl-4-ylmethyl)-4-formyl-2-methyl-pyrimidin [Smp.: 114-115° (aus Diisopropylether)].

c) Zu einer Lösung von 6-(n-Butyl)5-(2′-cyanobiphenyl-4-ylmethyl)-4-formyl-2-methyl-pyrimidin (8,8 g, 0,024 mol) in Methanol (135 ml) werden Natriumcyanid (5,81 g, 0,12 mol), aktiviertes Mangandioxid (0,474 mol) und Essigsäure (2,4 ml) zugegeben und die entstandene Suspension wird sodann 12 Stunden bei Raumtemperatur gerührt. Nach Filtration des Reaktionsgemisches wird das Filtrat im Vakuum eingedampft und der Rückstand zwischen Essigester und gesättigter $NaHCO_3$-Lösung verteilt. Die organische Phase wird mit gesättigter NaCl-Lösung gewaschen, getrocknet ($Na_2SO_4$) und im Vakuum eingedampft. Flash-Chromatographie (Kieselgel 60,40-63 µm, Hexan/Essigester = 2:1) ergibt 6-(n-Butyl)-5-(2′-cyanobiphenyl-4-ylmethyl)-4-methoxycarbonyl-2-methyl-pyrimidin, welches direkt weiterverarbeitet wird.

Beispiel 24:

In analoger Weise wie in einem der vorstehenden Beispiele beschrieben, kann man herstellen:

6-(n-Butyl)-4-ethyl-2-methyl-5-[2′-(1H-tetrazol-5-yl)biphenyl-4-ylmethyl]-pyrimidin,
6-(n-Butyl)-4-ethoxycarbonyl-2-methyl-5-[2′-(1H-tetrazol-5-yl)biphenyl-4-ylmethyl]-pyrimidin,
6-(n-Butyl)-2-(tert.-butyl)-4-hydroxy-5-[2′-(1H-tetrazol-5-yl)biphenyl-4-ylmethyl]-pyrimidin,
6-(n-Butyl)-4-mercapto-2-methyl-5-[2′-(1H-tetrazol-5-yl)biphenyl-4-ylmethyl]-pyrimidin,
6-(n-Butyl)-4-methansulfonyl-2-methyl-5-[2′-(1H-tetrazol-5-yl)biphenyl-4-ylmethyl]-pyrimidin,
6-(n-Butyl)-2-methyl-5-[2′-(1H-tetrazol-5-yl)biphenyl-4-ylmethyl]-pyrimidin-4-sulfonsäure,
6-(n-Butyl)-2-methyl-4-sulfamoyl-5-[2′-(1H-tetrazol-5-yl)biphenyl-4-ylmethyl]- pyrimidin,
6-(n-Butyl)-2-methyl-4-methylamino-5-[2′-(1H-tetrazol-5-yl)biphenyl-4-ylmethyl]-pyrimidin,
6-(n-Butyl)-4-methansulfonylamino-2-methyl-5-[2′-(1H-tetrazol-5-yl)biphenyl-4-ylmethyl]-pyrimidin,
4-Acetylamino-6-(n-butyl)-2-methyl-5-[2′-(1H-tetrazol-5-yl)biphenyl-4-ylmethyl]-pyrimidin,
6-(n-Butyl)-4-ethoxycarbonylmethyl-2-methyl-5-[2′-(1H-tetrazol-5-yl)biphenyl-4-ylmethyl]-pyrimidin,
6-(n-Butyl)-4-carboxymethyl-2-methyl-5-[2′-(1H-tetrazol-5-yl)biphenyl-4-ylmethyl]-pyrimidin,
6(n-Butyl)-4-hydroxymethyl-2-methyl-5-[2′-(1H-tetrazol-5-yl)biphenyl-4-ylmethyl]-pyrimidin,
6-(n-Butyl)-4-methoxymethyl-2-methyl-5-[2′-(1H-tetrazol-5-yl)biphenyl-4-ylmethyl]-pyrimidin,
6-(n-Butyl)-2,4-dimethyl-5-[2′-( 1H-tetrazol-5-yl)biphenyl-4-ylmethyl]-pyrimidin,
6-(n-Butyl)-2-methyl-5-[2′-(1H-tetrazol-5-yl)biphenyl-4-ylmethyl]-pyrimidin-4-phosphonsäure,
6-(n-Butyl)-2-methyl-4-(1H-tetrazol-5-yl)-5-[2′-(1H-tetrazol-5-yl)biphenyl-4-ylmethyl]-pyrimidin,
4-Hydroxy-2-methyl-5-[2′-(1H-tetrazol-5-yl)biphenyl-4-yloxy]-pyrimidin,
4-Hydroxy-2-methyl-5-[2′-(1H-tetrazol-5-yl)biphenyl-4-ylthio]-pyrimidin,
6-(n-Butyl)-4-carboxymethoxy-2-methyl-5-[2′-(1H-tetrazol-5-yl)biphenyl-4-ylmethyl]-pyrimidin,
6-(n-Butyl)-4-ethoxycarbonylmethylamino-2-methyl-5-[2′-(1H-tetrazol-5-yl)biphenyl-4-ylmethyl]-pyrimidin,
und

6-(n-Butyl)-4-carboxymethylamino-2-methyl-5-[2′-(1H-tetrazol-5-yl)biphenyl-4-ylmethyl]-pyrimidin.

Beispiel 25:

Tabletten, enthaltend je 50 mg Wirkstoff, z.B. 2,6-Di-(n-butyl)-4-hydroxy-5-[2′-(1H-tetrazol-5-yl)biphenyl-4-ylmethyl]-pyrimidin, können wie folgt hergestellt werden:

| Zusammensetzung (für 10000 Tabletten): | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 26:

Lacktabletten, enthaltend je 100 mg Wirkstoff, z.B. 2,6-Di-(n-butyl)-4-hydroxy-5-[2′-(1H-tetrazol-5-yl)-biphenyl-4-ylmethyl]-pyrimidin, können wie folgt hergestellt werden:

| Zusammensetzung (für 1000 Tabletten): | |
|---|---|
| Wirkstoff | 100,00 g |
| Lactose | 100,00 g |
| Maisstärke | 70,00 g |
| Talk | 8,50 g |
| Calciumstearat | 1,50 g |
| Hydroxypropylmethylcellulose | 2,36 g |
| Schellack | 0,64 g |
| Wasser | q.s. |
| Dichlormethan | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der

Hydroxypropylmethylcellulose und des Schellacks in Dichlormethan lackiert (Endgewicht der Lacktablette: 283 mg).

Beispiel 27:

In analoger Weise wie in den Beispielen 25 und 26 beschrieben können auch Tabletten und Lacktabletten, enthaltend eine andere Verbindung der Formel I oder ein Tautomeres und/oder ein pharmazeutisch verwendbares Salz einer Verbindung der Formel I, z.B. gemäss einem der Beispiele 1 bis 24, hergestellt werden.

**Ansprüche**

1. Eine Verbindung der Formel

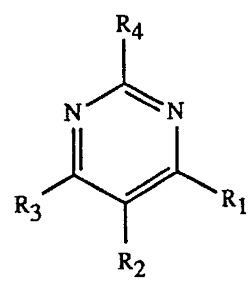

(I),

worin einer der Reste $R_1$, $R_2$ und $R_3$ einen gegebenenfalls durch Halogen oder Hydroxy substituierten aliphatischen Kohlenwasserstoffrest oder einen cycloaliphatischen oder araliphatischen Kohlenwasserstoffrest bedeutet, der zweite der Reste $R_1$, $R_2$ und $R_3$ und der Rest $R_4$ jeweils unabhängig voneinander Halogen, Acyl, einen aromatischen Kohlenwasserstoffrest, gegebenenfalls verestertes oder amidiertes Carboxy, Cyano, $SO_3H$, $PO_2H_2$, $PO_3H_2$, 5-Tetrazolyl, gegebenenfalls substituiertes Sulfamoyl, Acylamino oder $-Z_1-R'$, worin $Z_1$ für eine Bindung oder für O, $S(O)_m$ oder N(R) steht, R' Wasserstoff oder einen aliphatischen Kohlenwasserstoffrest bedeutet, der gegebenenfalls durch O oder $S(O)_m$ unterbrochen ist und gegebenenfalls durch Halogen, Hydroxy, gegebenenfalls substituiertes Amino oder gegebenenfalls verestertes oder amidiertes Carboxy substituiert ist, R Wasserstoff oder einen aliphatischen Kohlenwasserstoffrest bedeutet und m jeweils für 0, 1 oder 2 steht, bedeuten und der dritte der Reste $R_1$, $R_2$ und $R_3$ für die Gruppe der Formel

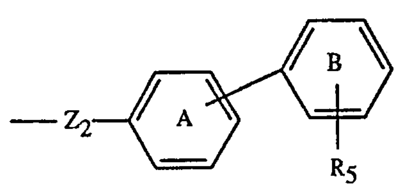

(Ia)

steht, worin $Z_2$ für Alkylen, O, $S(O)_m$ oder N(R) steht, $R_5$ Carboxy, Halogenalkansulfonylamino, $SO_3H$, $PO_2H_2$, $PO_3H_2$ oder 5-Tetrazolyl bedeutet, R Wasserstoff oder einen aliphatischen Kohlenwasserstoffrest bedeutet, m für 0,1 oder 2 steht und die Ringe A und B unabhängig voneinander gegebenenfalls durch Halogen, einen aliphatischen Kohlenwasserstoffrest, der gegebenenfalls durch O unterbrochen ist und gegebenenfalls durch Hydroxy oder Halogen substituiert ist, gegebenenfalls durch einen aliphatischen Alkohol verethertes Hydroxy, gegebenenfalls verestertes oder amidiertes Carboxy oder 5-Tetrazolyl substituiert sind, oder gegebenenfalls ein Tautomeres davon, jeweils in freier Form oder in Salzform.

2. Eine Verbindung gemäss Anspruch 1 der Formel I, worin einer der Reste $R_1$, $R_2$ und $R_3$ jeweils gegebenenfalls durch Halogen oder Hydroxy substituiertes Niederalkyl, Niederalkenyl oder Niederalkinyl, jeweils 3- bis 7-gliedriges Cycloalkyl oder Cycloalkenyl, Phenylniederalkyl, Phenylniederalkenyl oder Phenylniederalkinyl bedeutet, der zweite der Reste $R_1$, $R_2$ und $R_3$ und der Rest $R_4$ jeweils unabhängig voneinander Halogen, Niederalkanoyl, gegebenenfalls substituiertes Phenyl, Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder disubstituiert oder durch Niederalkylen oder Niederalkylenoxyniederalkylen disubstituiert ist, Cyano, $SO_3H$, $PO_2H_2$, $PO_3H_2$, 5-Tetrazolyl, Sulfamoyl, Niederalkansulfamoyl, Diniederalkansulfamoyl, Niederalkanoylamino, gegebenenfalls substituiertes Benzoylamino, Niederalkansulfonylamino, Halogenniederalkansulfonylamino, gegebenenfalls substituiertes Benzolsulfonylamino oder $-Z_1-R'$, worin $Z_1$ für eine Bindung oder für O, $S(O)_m$ oder N(R) steht, $R'$ Wasserstoff oder jeweils gegebenenfalls durch Hydroxy, Halogen, Amino, Niederalkylenamino, Niederalkylenoxyniederalkylenamino, Niederalkylamino, Niederalkenylamino, Niederalkinylamino, Phenylniederalkylamino, Phenylniederalkenylamino, Phenylniederalkinylamino, Diniederalkylamino, N-Niederalkyl-N-phenylniederalkyl-amino, Di(phenylniederalkyl)amino, Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, oder Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono-oder di-substituiert oder durch Niederalkylen oder Niederalkylenoxyniederalkylen disubstituiert ist, substituiertes Niederalkyl, Niederalkenyl, Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl, Niederalkenyloxyniederalkinyl, Niederalkylthio-niederalkyl, -niederalkenyl oder -niederalkinyl, Niederalkan-sulfinylniederalkyl oder -sulfonylniederalkyl, Niederalkenyl-thioniederalkyl, -sulfinylniederalkyl oder -sulfonylniederalkyl oder Niederalkinyl-thioniederalkyl, -sulfinylniederalkyl oder -sulfonylniederalkyl bedeutet, R Wasserstoff, Niederalkyl, Niederalkenyl oder Niederalkinyl bedeutet und m für 0,1 oder 2 steht, bedeuten und der dritte der Reste $R_1$, $R_2$ und $R_3$ für die Gruppe der Formel Ia steht, worin $Z_2$ für Methylen, Niederalkylen, O, $S(O)_m$ oder N(R) steht, $R_5$ Carboxy, Halogenalkansulfonylamino, $SO_3H$, $PO_2H_2$, $PO_3H_2$ oder 5-Tetrazolyl bedeutet, R Wasserstoff, Niederalkyl, Niederalkenyl oder Niederalkinyl bedeutet, m für 0,1 oder 2 steht und die Ringe A und B unabhängig voneinander gegebenenfalls durch Halogen, durch gegebenenfalls durch Hydroxy oder Halogen substituiertes Niederalkyl, Niederalkenyl, Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl oder Niederalkenyloxyniederalkinyl, durch Hydroxy, durch Niederalkoxy, durch Niederalkenyloxy, durch Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, durch Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder di-substituiert oder durch Niederalkylen oder Niederalkylenoxyniederalkylen disubstituiert ist, oder durch 5-Tetrazolyl substituiert sind, wobei die aromatischen Substituenten jeweils gegebenenfalls durch Niederalkyl, Niederalkoxy, Halogen, Trifluormethyl und/oder Hydroxy substituiert sind, oder gegebenenfalls ein Tautomeres davon, jeweils in freier Form oder in Salzform.

3. Eine Verbindung gemäss Anspruch 1 der Formel I, worin $R_1$ Halogen, Carboxy, welches gegebenenfalls durch einen Alkohol verestert ist, der sich von Niederalkyl oder Niederalkoxyniederalkyl ableitet, Carbamoyl, Cyano, $PO_3H_2$, 5-Tetrazolyl, Niederalkansulfamoyl, Niederalkanoylamino, Niederalkansulfonylamino oder $-Z_1-R'$, worin $Z_1$ für eine Bindung oder für O, $S(O)_m$ oder N(R) steht, $R'$ Wasserstoff oder jeweils gegebenenfalls durch Carboxy, Niederalkoxycarbonyl, Niederalkoxyniederalkoxycarbonyl oder Hydroxy substituiertes Niederalkyl oder Niederalkoxyniederalkyl bedeutet, R Wasserstoff oder Niederalkyl ist und m für 0,1 oder 2 steht, bedeutet, $R_2$ für die Gruppe der Formel Ia steht, worin $Z_2$ für Methylen, Niederalkylen, O, $S(O)_m$ oder N(R) steht, R Wasserstoff oder Niederalkyl bedeutet, m für 0,1 oder 2 steht, $R_5$ Carboxy oder 5-Tetrazolyl bedeutet und die Ringe A und B unabhängig voneinander gegebenenfalls durch Halogen, Niederalkyl, Niederalkoxy, Carboxy, Niederalkoxycarbonyl oder 5-Tetrazolyl substituiert sind, $R_3$ gegebenenfalls durch Hydroxy oder Halogen substituiertes Niederalkyl oder Niederalkenyl bedeutet und $R_4$ Niederalkyl bedeutet, oder gegebenenfalls ein Tautomeres davon, jeweils in freier Form oder in Salzform.

4. Eine Verbindung gemäss Anspruch 1 der Formel I, worin $R_1$ Halogen, insbesondere mit einer Atomnummer bis und mit 35, Carboxy, Niederalkoxycarbonyl, $PO_3H_2$, 5-Tetrazolyl, Niederalkanoylamino, Niederalkansulfonylamino,Wasserstoff, Niederalkyl, Niederalkoxyniederalkyl, Carboxyniederalkyl, Niederalkoxycarbonylniederalkyl, Hydroxyniederalkyl, Hydroxyniederalkoxyniederalkyl, Hydroxy, Niederalkoxy, Niederalkoxyniederalkoxy, Carboxyniederalkoxy, Niederalkoxycarbonylniederalkoxy, Hydroxyniederalkoxy, Mercapto, Niederalkylthio, Niederalkansulfinyl, Niederalkansulfonyl, Amino, Niederalkylaminooder Diniederalkylamino bedeutet, $R_2$ für die Gruppe der Formel Ia steht, worin $Z_2$ für Methylen oder Niederalkylen, O oder $S(O)_m$ steht, m für 0, 1 oder 2 steht, $R_5$ Carboxy oder 5-Tetrazolyl bedeutet und die Ringe A und B unabhängig voneinander gegebenenfalls durch Niederalkyl, Halogen, insbesondere mit einer Atomnummer bis und mit

35, oder Niederalkoxy substituiert sind, $R_3$ $C_1$-$C_7$-Alkyl bedeutet und $R_4$ Niederalkyl bedeutet, wobei mit "nieder" bezeichnete Teilstrukturen jeweils insbesondere bis und mit 7, vorzugsweise bis und mit 4, C-Atome umfassen, oder gegebenenfalls ein Tautomeres davon, jeweils in freier Form oder in Salzform.

5. Eine Verbindung gemäss einem der Ansprüche 1 bis 4 der Formel I, worin $R_2$ für die Gruppe der Formel

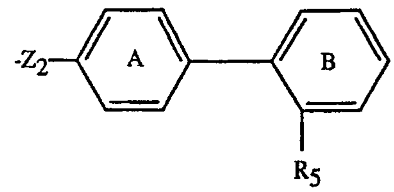

(Ib)

steht, oder gegebenenfalls ein Tautomeres davon, jeweils in freier Form oder in Salzform.

6. Eine Verbindung gemäss Anspruch 1 der Formel I, worin $R_1$ Halogen mit einer Atomnummer bis und mit 35, Hydroxy, Niederalkoxy mit bis und mit 4C-Atomen, Niederalkoxyniederalkoxy mit bis und mit 4C-Atomen in jedem Niederalkoxyteil, Hydroxyniederalkoxy mit bis und mit 4C-Atomen, Niederalkylamino mit bis und mit 4 C-Atomen oder Diniederalkylamino mit bis und mit 4 C-Atomen in jedem Niederalkylteil bedeutet, $R_2$ für die Gruppe der Formel Ib steht, worin $Z_2$ Methylen bedeutet, $R_5$ Carboxy oder insbesondere 5-Tetrazolyl bedeutet und die Ringe A und B unsubstituiert sind, $R_3$ $C_1$-$C_7$-Alkyl, insbesondere $C_1$-$C_5$-Alkyl bedeutet und $R_4$ Niederalkyl mit bis und mit 4 C-Atomen bedeutet, oder gegebenenfalls ein Tautomeres davon, jeweils in freier Form oder in Salzform.

7. Eine Verbindung gemäss Anspruch 1 der Formel I, worin $R_1$ Hydroxy bedeutet, $R_2$ für die Gruppe der Formel Ib steht, worin $Z_2$ Methylen bedeutet, $R_5$ 5-Tetrazolyl bedeutet und die Ringe A und B unsubstituiert sind, $R_3$ $C_1$-$C_5$-Alkyl bedeutet und $R_4$ Niederalkyl mit bis und mit 4 C-Atomen bedeutet, oder gegebenenfalls ein Tautomeres davon, jeweils in freier Form oder in Salzform.

8. 6(n-Butyl)-4-hydroxy-2-methyl-5-[2'-(1H-tetrazol-5-yl)biphenyl4-ylmethyl]-pyrimidin oder ein Tautomeres und/oder Salz davon.

9. 6-(n-Butyl)-4-hydroxy-2-(n-propyl)-5-[2'-(1H-tetrazol-5-yl)biphenyl-4-ylmethyl]-pyrimidin oder ein Tautomeres und/oder Salz davon.

10. 2,6-Di-(n-butyl)-4-hydroxy-5-[2'-(1H-tetrazol-5-yl)biphenyl-4-ylmethyl]-pyrimidin oder ein Tautomeres und/oder Salz davon.

11. 6-(n-Butyl)-4-hydroxy-2-isopropyl-5-[2'-( 1H-tetrazol-5-yl)biphenyl-4-ylmethyl]-pyrimidin oder ein Tautomeres und/oder Salz davon.

12. 6-(n-Butyl)-2-ethyl-4-hydroxy-5-[2'-(1H-tetrazol-5-yl)biphenyl-4-ylmethyl]-pyrimidin oder ein Tautomeres und/oder Salz davon.

13. 4-Hydroxy-2-methyl-6-(n-propyl)-5-[2'-(1H-tetrazol-5-yl)biphenyl-4-ylmethyl]-pyrimidin,
6-(n-Butyl)-4-chlor-2-methyl-5-[2'-(1H-tetrazol-5-yl)biphenyl4-ylmethyl]-pyrimidin,
6-(n-Butyl)-4-methoxy-2-methyl-5-[2'-(1H-tetrazol-5-yl)biphenyl-4-ylmethyl]-pyrimidin,
6-(n-Butyl)-4-(2-methoxyethoxy)-2-methyl--5-[2'-(1H-tetrazol-5-yl)biphenyl-4-ylmethyl]-pyrimidin,
6-(n-Butyl)-4-dimethylamino-2-methyl-5-[2'-( 1H-tetrazol-5-yl)biphenyl-4-ylmethyl]-pyrimidin,
4-Amino-6-(n-butyl)-2-methyl-5-[2'-( 1H-tetrazol-5-yl)biphenyl-4-ylmethyl]-pyrimidin,
6-(n-Butyl)-2-methyl-4-methylthio--5-[2'-(1H-tetrazol-5-yl)biphenyl-4-ylmethyl]-pyrimidin,
6-(n-Butyl)-4-(2-hydroxyethoxy)-2-methyl-5-[2'-(1H-tetrazol-5-yl)biphenyl-4-ylmethyl]-pyrimidin,
6-(n-Butyl)-4-ethoxycarbonylmethoxy-2-methyl-5-[2'-(1H-tetrazol-5-yl)biphenyl-4-ylmethyl]-pyrimidin,
6-(n-Butyl)-4-carboxy-2-methyl-5-[2'-( 1H-tetrazol-5-yl)biphenyl-4-ylmethyl]-pyrimidin,
6-(n-Butyl)-4-ethoxycarbonyl-2-methyl-5- [2'-(1H-tetrazol-5-yl)biphenyl-4-ylmethyl]-pyrimidin,
6-(n-Butyl)-2-(tert.-butyl)-4-hydroxy-5-[2'-(1H-tetrazol-5-yl)biphenyl-4-ylmethyl]-pyrimidin,
6-(n-Butyl)-4-mercapto-2-methyl-5-[2'-(1H-tetrazol-5-yl)biphenyl-4-ylmethyl]-pyrimidin,
6-(n-Butyl)-4-methansulfonyl-2-methyl-5-[2'-(1H-tetrazol-5-yl)biphenyl-4-ylmethyl]-pyrimidin,
6-(n-Butyl)-2-methyl-5-[2'-( 1H-tetrazol-5-yl)biphenyl-4-ylmethyl]-pyrimidine-4-sulfonsäure,
6-(n-Butyl)-2-methyl-4-sulfamoyl-5-[2'-(1H-tetrazol-5-yl)biphenyl-4-ylmethyl]-pyrimidin,
6-(n-Butyl)-2-methyl-4-methylamino-5-[2'-(1H-tetrazol-5-yl)biphenyl-4-ylmethyl]-pyrimidin,
6-(n-Butyl)-4-methansulfonylamino-2-methyl-5-[2'-(1H-tetrazol-5-yl)biphenyl-4-ylmethyl]-pyrimidin,
4-Acetylamino-6-(n-butyl)-2-methyl-5-[2'-(1H-tetrazol-5-yl)biphenyl-4-ylmethyl]-pyrimidin,
6-(n-Butyl)-4-ethoxycarbonylmethyl-2-methyl-5-[2'-(1H-tetrazol-5-yl)biphenyl-4-ylmethyl]-pyrimidin,
6-(n-Butyl)-4-carboxymethyl-2-methyl-5-[2'-(1H-tetrazol-5-yl)biphenyl-4-ylmethyl]-pyrimidin,
6-(n-Butyl)-4-hydroxymethyl-2-methyl-5-[2'-(1H-tetrazol-5-yl)biphenyl-4-ylmethyl]-pyrimidin,

6-(n-Butyl)-4-methoxymethyl-2-methyl-5-[2'-(1H-tetrazol-5-yl)biphenyl-4-ylmethyl]-pyrimidin,
6-(n-Butyl)-2,4-dimethyl-5-[2'-(1H-tetrazol-5-yl)biphenyl-4-ylmethyl]-pyrimidin,
6-(n-Butyl)-2-methyl-5-[2'-( 1H-tetrazol-5-yl)biphenyl-4-ylmethyl]-pyrimidine-4-phosphonsäure,
6-(n-Butyl)-2-methyl-4-(1H-tetrazol-5-yl)-5-[2' -(1H-tetrazol-5-yl)biphenyl-4-ylmethyl]-pyrimidin,
4-Hydroxy-2-methyl-5-[2'-(1H-tetrazol-5-yl)biphenyl-4-yloxy]-pyrimidin,
4-Hydroxy-2-methyl-5-[2'-(1H tetrazol-5-yl)biphenyl-4-ylthio]-pyrimidin,
6-(n-Butyl)-4-carboxymethoxy-2-methyl-5-[2'-(1H tetrazol-5-yl)biphenyl-4-ylmethyl]- pyrimidin,
6-(n-Butyl)-4-ethoxycarbonylmethylamino-2-methyl-5-[2'-(1H-tetrazol-5-yl)biphenyl-4-ylmethyl]-pyrimidin oder
6-(n-Butyl)-4-carboxymethylamino-2-methyl-5-[2'-(1H-tetrazol-5-yl)biphenyl-4-ylmethyl]-pyrimidin, oder jeweils ein Tautomeres und/oder Salz davon.

14. 6-(n-Butyl)-4-hydroxy-5-[2'-(1H-tetrazol-5-yl)biphenyl-4-ylmethyl]-pyrimidin oder ein Tautomeres und/oder Salz davon.

15. 6-(n-Butyl)-4-ethyl-2-methyl-5-[2'-(1H-tetrazol-5-yl)biphenyl-4-ylmethyl]-pyrimidin,
6-(But-1-en-4-yl)-2-(n-butyl)-4-hydroxy-5-[2'-(1H-tetrazol-5-yl)biphenyl-4-ylmethyl]-pyrimidin,
5-[3-Brom-2'-(1H-tetrazol-5-yl)-biphenyl-4-ylmethyl]-2,6-di-(n-butyl)-4-hydroxy-pyrimidin,
2,6-Di-(n-butyl)-4-hydroxy-5-[3'-(1H-tetrazol-5-yl)biphenyl-4-ylmethyl]-pyrimidin,
2,6-Di-(n-butyl)-4-hydroxy-5-[2'-(1H-tetrazol-5-yl)biphenyl-3-ylmethyl]-pyrimidin oder
2,6-Di-(n-butyl)-4-hydroxy-5-[2-(2'-(1H-tetrazol-5-yl)biphenyl-4-yl)ethyl]-pyrimidin,
oder jeweils ein Tautomeres und/oder Salz davon.

16. Eine Verbindung gemäss einem der Ansprüche 1 bis 15 oder ein Tautomeres davon, jeweils in freier Form oder in Form eines pharmazeutisch verwendbaren Salzes, zur Anwendung in einem Verfahren zur therapeutischen Behandlung des menschlichen oder tierischen Körpers.

17. Eine Verbindung gemäss einem der Ansprüche 4,5 und 8 bis 13 oder ein Tautomeres davon, jeweils in freier Form oder in Form eines pharmazeutisch verwendbaren Salzes, zur Anwendung in einem Verfahren zur therapeutischen Behandlung des menschlichen oder tierischen Körpers.

18. Eine Verbindung gemäss einem der Ansprüche 1 bis 17 oder ein Tautomeres davon, jeweils in freier Form oder in Form eines pharmazeutisch verwendbaren Salzes, zur Anwendung als Antihypertensivum.

19. Eine Verbindung gemäss einem der Ansprüche 4,5,8 bis 13 und 17 oder ein Tautomeres davon, jeweils in freier Form oder in Form eines pharmazeutisch verwendbaren Salzes, zur Anwendung als Antihypertensivum.

20. Ein pharmazeutisches Präparat, als Wirkstoff enthaltend eine Verbindung gemäss einem der Ansprüche 1 bis 19 oder ein Tautomeres davon, jeweils in freier Form oder in Form eines pharmazeutisch verwendbaren Salzes, gegebenenfalls neben üblichen pharmazeutischen Kilfsstoffen.

21. Ein pharmazeutisches Präparat, als Wirkstoff enthaltend eine Verbindung gemäss einem der Ansprüche 4,5,8 bis 13,17 und 19 oder ein Tautomeres davon, jeweils in freier Form oder in Form eines pharmazeutisch verwendbaren Salzes, gegebenenfalls neben üblichen pharmazeutischen Hilfsstoffen.

22. Ein antihypertensiv wirksames pharmazeutisches Präparat gemäss Anspruch 20 oder 21, dadurch gekennzeichnet, dass man einen antihypertensiv wirksamen Wirkstoff wählt.

23. Ein antihypertensiv wirksames pharmazeutisches Präparat gemäss Anspruch 21, dadurch gekennzeichnet, dass man einen antihypertensiv wirksamen Wirkstoff wählt.

24. Verfahren zur Herstellung einer Verbindung gemäss einem der Ansprüche 1 bis 15 der Formel I oder gegebenenfalls eines Tautomeren davon, jeweils in freier Form oder in Salzform, dadurch gekennzeichnet, dass man

a) in einer Verbindung der Formel

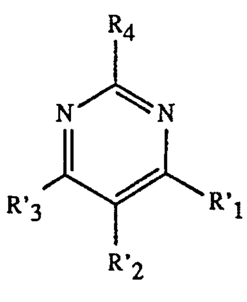

(IIa)

oder einem Tautomeren und/oder Salz davon, worin einer der Reste $R'_1$, $R'_2$ und $R'_3$ für die Gruppe der Formel

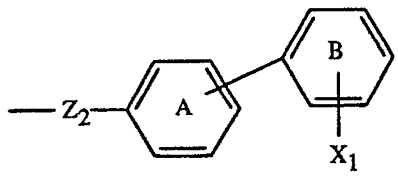

(IIb)

steht, worin $X_1$ ein in $R_5$ überführbaren Rest ist, und die beiden anderen Reste die von der Gruppe der Formel Ia verschiedenen Bedeutungen von $R_1$, $R_2$ bzw. $R_3$ haben, $X_1$ in $R_5$ überführt oder

b) eine Verbindung der Formel

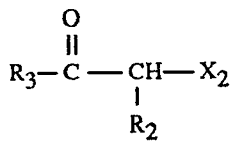

(IIIa),

worin $X_2$ gegebenenfalls funktionell abgewandeltes Carboxy bedeutet, mit einer Verbindung der Formel

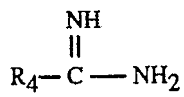

(IIIb)

oder einem Salz davon umsetzt und jeweils, wenn erwünscht, eine verfahrensgemäss oder auf andere Weise erhältliche Verbindung der Formel I oder ein Tautomeres davon, jeweils in freier Form oder in Salzform, in eine andere Verbindung der Formel I oder ein Tautomeres davon überführt, ein verfahrensgemäss erhältliches Gemisch von Isomeren auftrennt und das gewünschte Isomere isoliert und/oder eine verfahrensgemäss erhältliche freie Verbindung der Formel I oder ein Tautomeres davon in ein Salz oder ein verfahrensgemäss erhältliches Salz einer Verbindung der Formel I oder eines Tautomeren davon in die freie Verbindung der Formel I oder ein Tautomeres davon oder in ein anderes Salz überführt.

25. Verfahren zur Herstellung eines pharmazeutischen Präparats gemäss einem der Ansprüche 20 bis 23, dadurch gekennzeichnet, dass man den Wirkstoff, gegebenenfalls unter Beimischung von üblichen pharmazeutischen Hilfsstoffen, zu einem pharmazeutischen Präparat verarbeitet.

26. Verfahren gemäss Anspruch 25 zur Herstellung eines antihypertensiv wirksamen pharmazeutischen Präparats gemäss Anspruch 22 oder 23, dadurch gekennzeichnet, dass man einen antihypertensiv wirksamen Wirkstoff wählt.

27. Verwendung einer Verbindung gemäss einem der Ansprüche 1 bis 19 oder gegebenenfalls eines Tautomeren davon, jeweils in freier Form oder in Form eines pharmazeutisch verwendbaren Salzes, zur Herstellung eines pharmazeutischen Präparats.

28. Verwendung einer Verbindung gemäss einem der Ansprüche 1 bis 19 oder gegebenenfalls eines Tautomeren davon, jeweils in freier Form oder in Form eines pharmazeutisch verwendbaren Salzes, zur Herstellung eines pharmazeutischen Präparats auf nicht-chemischem Wege.

29. Verwendung einer Verbindung gemäss Anspruch 27 oder 28 zur Herstellung eines Antihypertensivums.